(19) **Europäisches Patentamt / European Patent Office / Office européen des brevets**

(11) **EP 2 198 774 B1**

(12) **EUROPEAN PATENT SPECIFICATION**

(45) Date of publication and mention
of the grant of the patent:
**26.09.2018 Bulletin 2018/39**

(21) Application number: **09015799.1**

(22) Date of filing: **21.12.2009**

(51) Int Cl.:
*A61B 5/00* (2006.01)          *A61B 5/145* (2006.01)
*A61B 5/1486* (2006.01)

(54) **In vivo component measurement method and in vivo component measurement apparatus**

In-vivo-Komponentenmessverfahren und in-vivo-Komponentenmessgerät

Procédé de mesure de composant in vivo et appareil de mesure de composant in vivo

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **22.12.2008 JP 2008325824**
**26.11.2009 JP 2009269052**

(43) Date of publication of application:
**23.06.2010 Bulletin 2010/25**

(73) Proprietor: **SYSMEX CORPORATION**
**Kobe-shi,**
**Hyogo 651-0073 (JP)**

(72) Inventors:
• **Okada, Seiki**
  **Kobe-shi,**
  **Hyogo 6510073 (JP)**
• **Asakura, Yoshihiro**
  **Kobe-shi,**
  **Hyogo 6510073 (JP)**

• **Sato, Toshiyuki**
  **Kobe-shi,**
  **Hyogo 6510073 (JP)**
• **Hagino, Kei**
  **Kobe-shi,**
  **Hyogo 6510073 (JP)**
• **Kojima, Junko**
  **Kobe-shi,**
  **Hyogo 6510073 (JP)**
• **Kikkawa, Yasuo**
  **Kobe-shi,**
  **Hyogo 6510073 (JP)**

(74) Representative: **Hoffmann Eitle**
**Patent- und Rechtsanwälte PartmbB**
**Arabellastraße 30**
**81925 München (DE)**

(56) References cited:
EP-A1- 1 185 321          EP-A1- 1 270 041
EP-A1- 1 849 405          EP-A2- 1 964 512
WO-A2-03/011131          US-A1- 2004 087 671
US-A1- 2007 027 383      US-A1- 2007 232 875

**Description**

BACKGROUND OF THE INVENTION

Field of the Invention

The present invention relates to an in vivo component measurement method and an apparatus thereof.

Related Art

Conventionally, there has been known an in vivo component analyzer in which tissue fluid is extracted from a subject through a skin thereof, and glucose extracted with the tissue fluid is reacted to perform analysis by using enzyme such as glucose oxidase as a catalyst. For example, PCT Publication No. WO9502357 discloses an apparatus in which a collection device having a reservoir which includes a glucose collection medium made of water is arranged to a stratum corneum of the patient's skin for specific time (5 to 10 minutes), the glucose collection medium is taken out from the reservoir after specific time, and the glucose concentration is analyzed.

Since the quantity of tissue fluid thus extracted changes depending on skin states of the subjects, it is necessary to consider skin states of the subjects in order to measure a precise quantity of glucose. The apparatus described in WO9502357, however, does not at all consider such skin states of the subjects on measuring the quantity of glucose. In order to solve the above problem, it is proposed that glucose permeability (P) in the extracted site of the subject is predicted and a blood glucose level is calculated with computation formula (BG = J/P, where BG represents blood glucose level and J represents extracted glucose quantity) (Refer to US Publication No. US20070232875) .

Prediction principle of the glucose permeability (P) in US20070232875 is described below. It is known that electrolyte concentration in the tissue fluid is substantially similar among plural subjects having different blood glucose levels. For that reason, it is possible to predict a degree of tissue fluid permeating the skin (i.e. glucose permeability (P)) by measuring the electrolyte quantity which is included in the tissue fluid extracted through the skin. Therefore, pure water containing no electrolyte is used as an extraction medium holding the extracted tissue fluid, electricity is supplied to the extraction medium with the tissue fluid extracted, and electric conductivity (K) is measured so that the electrolyte quantity included in the extracted tissue fluid can be predicted. In other words, it is possible to predict the glucose permeability (P) from the electric conductivity (K) of the electrolyte of the extraction medium with the tissue fluid extracted. EP1964512 A2 is concerned with a method of measuring skin conductance, a method of analysing component concentration, a skin conductive measuring apparatus and a component concentration analyzer. US 2007/027383 A1 discloses measuring blood glucose and ion concentrations. It fails to disclose forming fine pores on skin.

As stated above, there have been desired developments of a further method for accurately analyzing the quantity of in vivo component such as glucose contained in the tissue fluid extracted from a biological body.

That is, it is an object of the present invention to provide an in vivo component measurement method and an apparatus thereof, capable of analyzing the quantity of in vivo component contained in the tissue fluid extracted from a biological body with a more accuracy than the conventional method.

SUMMARY OF THE INVENTION

As a result that the inventors studied hard to further improve the measurement accuracy of objective component such as glucose in the tissue fluid, particularly the measurement accuracy of area under the blood concentration time curve (AUC), they found that quantity of inorganic ion such as sodium ion, potassium ion and chloride ion are highly correlated with the extraction quantity of the objective component and accomplished success of the present invention.

That is, the in vivo component, particularly a value corresponding to a blood glucose AUC of the subject can be measured with a higher accuracy by focusing on the inorganic ion such as sodium ion, potassium ion and chloride ion, which was high correlativity with extraction quantity of the objective component, and by acquiring permeability of the objective component based on quantity of inorganic ion which is extracted.

An in vivo component measurement method (hereinafter simply referred to as "measurement method") according to a first aspect of the present invention is defined in claim 1.

In the measurement method according to the first aspect of the present invention, a permeability indicative of easiness to be extracted for the objective component such as glucose in the tissue fluid is obtained by using the reference value on the quantity of the inorganic ion which is highly correlated with a glucose permeability, and the extraction quantity of the objective component is predicted from the permeability thus obtained and the component information on the quantity of the objective component. Therefore, it is possible to acquire the value, for example a blood glucose AUC, on the quantity of the objective component at further high accuracy.

An in vivo component measurement apparatus(hereinafter simply referred to as "measurement apparatus") according to a second aspect of the present invention is defined in claim 6.

According to the in vivo component measurement method, and the in vivo component measurement apparatus of the present invention, it is possible to improve accuracy of the component measurement of the extracted tissue fluid. Multiple examples and embodiments are laid out in the present text. The scope of protection is defined in the appended claims.

BRIEF DESCRIPTION OF THE DRAWINGS

FIG. 1 is a schematic perspective view of a measurement apparatus, a sensor chip, and a collection member which are used for a blood glucose AUC measurement method according to a first embodiment of the present invention;
FIG. 2 is an explanatory plan view of the measurement apparatus shown in FIG. 1;
FIG. 3 is an explanatory side view of the measurement apparatus shown in FIG. 1;
FIG. 4 is an explanatory plan view of a sensor chip shown in FIG. 1;
FIG. 5 is an explanatory side view of the sensor chip shown in FIG. 1;
FIG. 6 is an explanatory sectional view of a collection member shown in FIG. 1;
FIG. 7 is an explanatory perspective view of an example of a puncture device used for a measurement method of the present invention;
FIG. 8 is a perspective view of a fine needle chip mounted on the puncture device shown in FIG. 7;
FIG. 9 is an explanatory sectional view of skin having the fine pore formed thereon with the puncture device;
FIG. 10 is a flowchart showing a measurement procedure of a blood glucose AUC measurement method according to first embodiment of the present invention;
FIG. 11 is an explanatory view of the measurement procedure of the blood glucose AUC measurement method according to the first embodiment of the present invention;
FIG. 12 is an explanatory view of the measurement procedure of the blood glucose AUC measurement method according to the first embodiment of the present invention;
FIG. 13 is an explanatory view of the measurement procedure of the blood glucose AUC measurement method according to the first embodiment of the present invention;
FIG. 14 is an explanatory sectional view of a reservoir member used for the blood glucose AUC measurement method according to second embodiment of the present invention;
FIG. 15 is an explanatory view of a measurement procedure of the blood glucose AUC measurement method according to the second embodiment of the present invention;
FIG. 16 is a view showing relation between blood glucose AUC and extraction glucose quantity;
FIG. 17 is a view showing relation between glucose permeability and ion extraction rate of sodium ion;
FIG. 18 is a view showing relation between glucose permeability and solvent conductivity;
FIG. 19 is a view showing relation between blood drawing blood glucose AUC and predicted blood glucose AUC in a case where the ion extraction rate of the sodium ion is used as a parameter;
FIG. 20 is a view showing relation between blood drawing blood glucose AUC and predicted blood glucose AUC in a case where the solvent conductivity is used as a parameter;
FIG. 21 is a view showing distribution of measurement differences of blood glucose AUC in a case where the ion extraction rate of the sodium ion is used as a parameter;
FIG. 22 is a view showing distribution of measurement differences of blood glucose AUC in a case where the solvent conductivity is used as a parameter;
FIG. 23 is a view showing relation between rate of the sodium ion contributing to the solvent conductivity and $\underline{r}$;
FIG. 24 is a graph showing correlation between $AUC_{BG}1h$ and extraction glucose quantity;
FIG. 25 is a graph showing correlation between $AUC_{BG}2h$ and extraction glucose quantity;
FIG. 26 is a graph showing correlation between glucose permeability and ion extraction rate of sodium ion (1 hour);
FIG. 27 is a graph showing correlation between glucose permeability and ion extraction rate of sodium ion (2 hours);
FIG. 28 is a graph showing correlation between blood drawing $AUC_{BG}1h$ and predicted $AUC_{BG}1h$;
FIG. 29 is a graph showing correlation between blood drawing $AUC_{BG}2h$ and predicted $AUC_{BG}2h$;
FIG. 30 is a view explaining an example of a method of collecting analyte from gel;
FIG. 31 is a view explaining an example of a method of collecting analyte from gel;
FIG. 32 is a view explaining another example of a method of collecting analyte from gel;
FIG. 33 is a view explaining another example of a method of collecting analyte from gel;
FIG. 34 is a view explaining another example of a method of collecting analyte from gel;
FIG. 35 is a view showing relation between glucose permeability and ion extraction rate of chloride ion; and
FIG 36 is a view showing relation between glucose permeability and ion extraction rate of potassium ion.

DETAILED DESCRIPTION

Hereinafter, embodiments of the measurement method and the measurement apparatus of the present invention are explained in detail with reference to figures attached hereto.

In the embodiments below, examples where the present invention is applied to measurement of blood glucose AUC are described. The blood glucose AUC refers to an area (unit: mg-h/dl) of a portion which is enclosed with a horizontal axis and a curve described by a graph representing time lapse of a blood glucose level. The blood glucose AUC is an index used for effect judgment of insulin and oral drugs in medical treatments of diabetes. For example, a value reflecting a total quantity of glucose (blood glucose) circulating in the blood within specific period after sugar tolerance (after meal) is measured by the blood glucose AUC so that a total quantity of glucose circulating in the biological body of the subject after sugar tolerance can be predicted.

Thus, significance of measuring the blood glucose AUC is that it is possible to control influences of personal differences in glucose metabolism. In other words, because there are personal differences in time required for a response to sugar tolerance to appear in the blood glucose level, it is difficult to grasp whether the blood glucose level is in rise time or in peak time, just by measuring the blood glucose level at a certain time after the sugar tolerance. Further, even if it is possible to measure the blood glucose level at the peak time, it is impossible to grasp how long a high blood, glucose state continues. In this respect, with the-blood glucose AUC measurement, it is possible to obtain a value reflecting a total quantity of blood glucose circulating in the blood within a specific period. Therefore the measurement value is not affected by time required for a response to sugar tolerance to appear in the blood glucose level, and further it is possible to predict how long the high blood glucose state continues based on the measurement value. Thus, it is possible to obtain a value useful for prediction of glucose tolerance due to sugar tolerance by measuring the blood glucose AUC, without influence of personal differences in glucose metabolism.

For measuring the blood glucose AUC, ordinarily, the blood is drawn every specific time (e.g. every 30 minutes) and blood glucose levels of the drawn blood are obtained respectively. Subsequently, a graph representing time lapse of the blood glucose level is obtained and an area of a portion enclosed with a horizontal axis and a curve described by the graph is obtained so that the blood glucose AUC is obtained. A value obtained using the blood glucose AUC measurement method according to the embodiment below is available for a judgment of diabetes instead of the blood glucose AUC by such blood drawing.

[First embodiment]

First, a measurement apparatatus, a sensor chip, and a collection member which are used for a blood glucose AUC measurement method according to a first embodiment of the present invention are described.

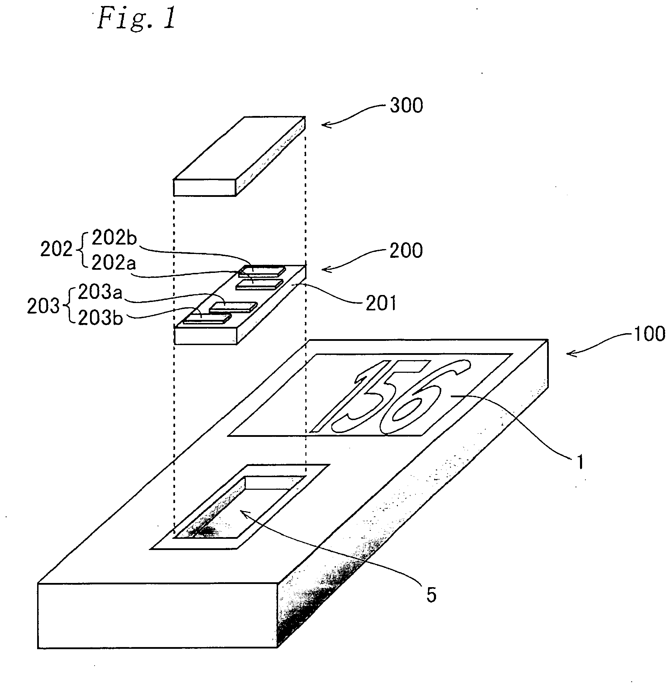

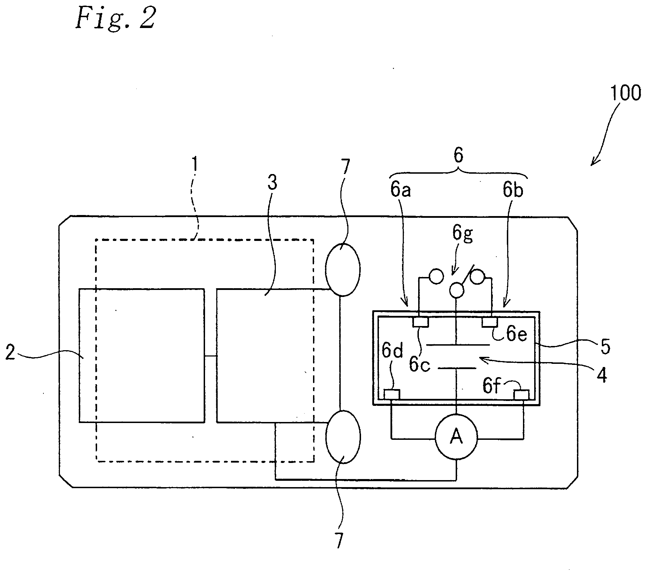

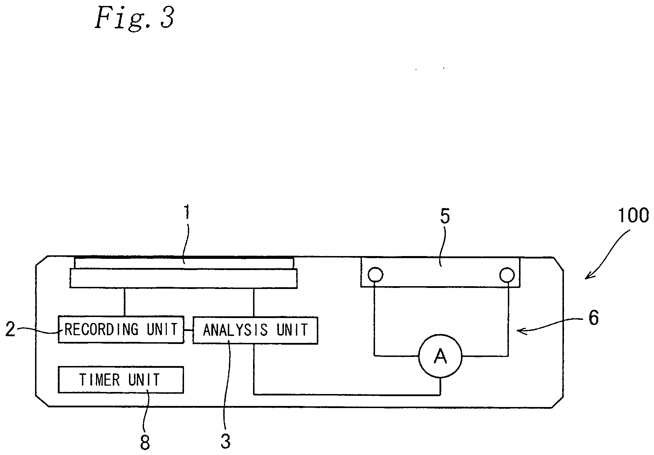

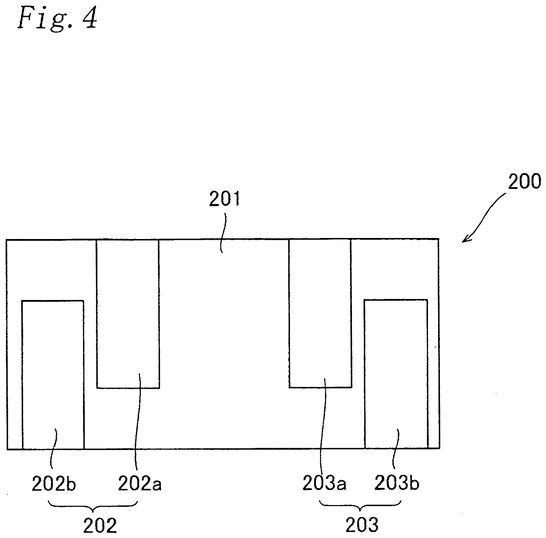

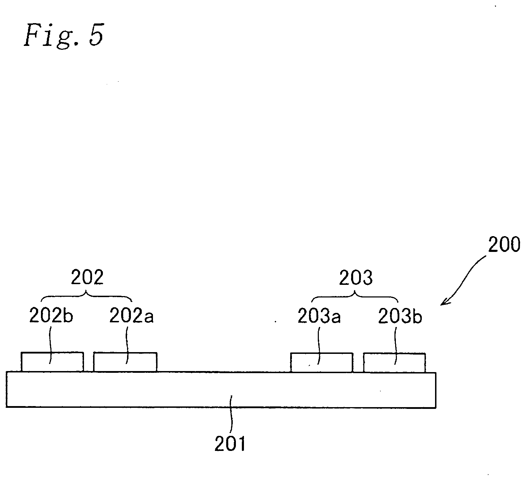

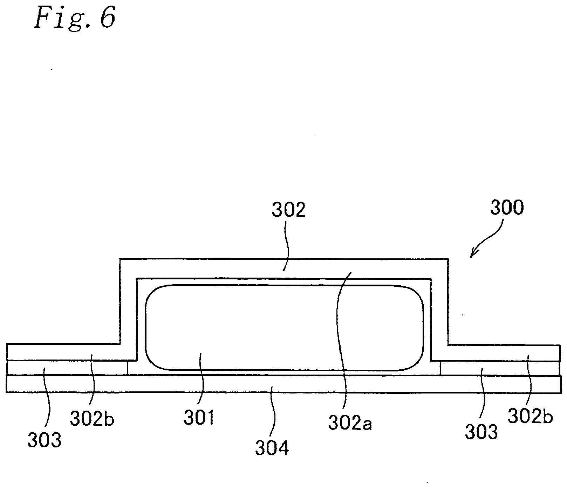

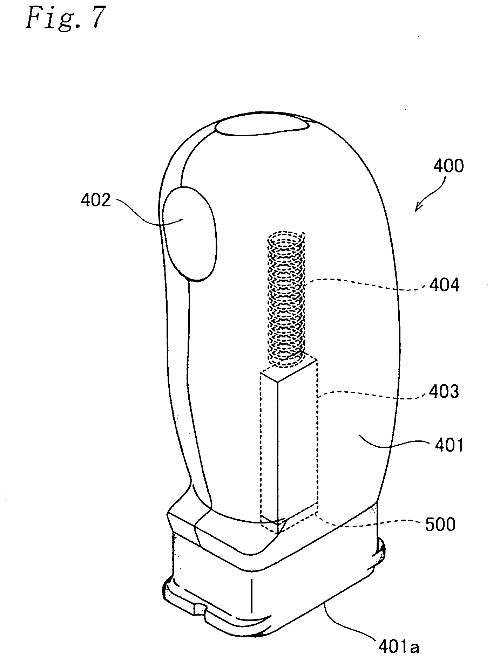

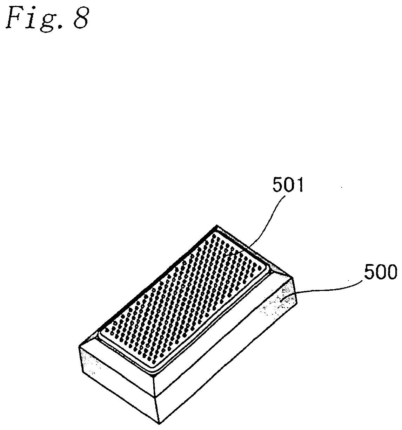

FIG. 1 is a schematic perspective view of a measurement apparatus, a sensor chip, and a collection member which are used for a blood glucose AUC measurement method according to a first embodiment of the present invention. FIGs. 2 and 3 are an explanatory plan view and an explanatory side view, respectively, of the measurement apparatus shown in FIG. 1. FIGs. 4 and 5 are an explanatory plan view and an explanatory side view, respectively, of a sensor chip shown in FIG. 1. FIG. 6 is an explanatory sectional view of a collection member shown in FIG. 1. FIG. 7 is an explanatory perspective view of an example of a puncture device used for the measurement method of the present invention. FIG. 8 is a perspective view of a fine needle chip mounted on the puncture device.

<Measurement system>

As shown in FIGs. 1 to 3, a measurement apparatus 100 comprises a display unit 1, a recording unit 2, an analysis unit 3, a power supply 4, an installation unit 5 being a setting unit for installing a sensor chip 200 and a collection member 300, an electric circuit 6 connected to the sensor chip 200 installed in the installation unit 5, an operation button 7 for a user (subject) to operate the measurement apparatus 100, and a timer 8.

The display unit 1 has a function of displaying a measurement result by the analysis unit 3 and data recorded in the recording unit 2. The recording unit 2 is provided for storing past data. The analysis unit 3 has a function of calculating a glucose concentration, and a concentration of inorganic ion such as sodium ion, potassium ion and chloride ion based on an output value of the electric circuit 6. The installation unit 5 has a concave shape and configured in such a manner that the sensor chip 200 and the collection member 300 are enabled to be installed. The electric circuit 6 includes a glucose concentration measurement circuit 6a and an ion concentration measurement circuit 6b. The glucose concentration measurement - circuit 6a includes terminals 6c and 6d which are exposed in the installation unit 5. The ion concentration measurement circuit 6b includes terminals 6e and 6f which are exposed in the installation unit 5. The electric circuit 6 includes a switch 6g for switching the glucose concentration measurement circuit 6a and the ion concentration measurement circuit 6b. The user can switch the glucose concentration measurement circuit 6a and the ion concentration measurement circuit 6b by operating the operation button 7 to operate the switch 6g. The operation button 7 is provided for switching the switch 6g, switching display in the display unit 1, and operating setting of the timer unit 8. The timer unit 8 has a function (function as a time information means) of informing extraction end time to the user for finishing extraction in specific time from the start of glucose extraction, and has an alarm device (not shown) built-in for that purpose.

<Sensor chip>

As shown in FIGs. 4 and 5, the sensor chip 200 comprises a substrate 201 made of synthetic resin, a pair of glucose concentration measurement electrodes 202 arranged on an upper surface of the substrate 201 and a pair of ion concentration measurement electrodes 203 arranged on the upper surface of the substrate 201. The glucose concentration measurement electrode 202 consists of a work electrode 202a with a GOD enzyme membrane (GOD: glucose oxidase) formed on a platinum electrode and a counter electrode 202b formed of a platinum electrode. On the other hand, the ion concentration measurement electrode 203 consists of an ion selective electrode 203a which is made of silver/silver chloride and has a selection membrane for inorganic ion, and a silver/silver chloride electrode 203b being a counter electrode. The work electrode 202a and the counter electrode 202b of the glucose concentration measurement electrode 202 respectively contact with the terminals 6c and 6d of the glucose concentration measurement circuit 6a, in a state that the sensor chip 200 is installed in the installation unit 5 of the measurement apparatus 100. Similarly, the ion selective electrode 203a and the silver/silver chloride electrode 203b of the ion concentration measurement electrode 203 respectively contact with the terminals 6e and 6f of the ion concentration measurement circuit 6b, in a state that the sensor chip 200 is installed in the installation unit 5 of the measurement system 100.

<Collection member>

As shown in FIG. 6, in a structure of the collection member 300, a gel 301 having moisture (substantially containing no $Na^+$) capable of retaining the tissue fluid extracted from the patent's skin is supported by a support member 302. The gel 301 in the present embodiment is made of polyvinyl alcohol and contains pure water as an extraction medium.

The support member 302 has a support main body 302a having a concave portion and a flange portion 302b formed in outer periphery of the support main body 302a, and the gel 301 is held inside the concave portion of the support main body 302a. An adhesive layer 303 is formed on a surface of the flange portion 302b, and a peel-off paper 304 for sealing the gel 301 held in the concave portion is applied to the adhesive layer 303 in a premeasurement state. During measurement, the adhesive layer 303 is removed from the peel-off paper 304, the gel 301 and the adhesive layer 303 are exposed, and the collection member 300 is enabled to be applied and fixed to the subject's skin through the adhesive layer 303 in a state that the gel 301 contacts to the subject's skin.

<Puncture device>

As shown in FIGs. 7 to 9, a puncture device 400 is a device which is mounted with a fine needle chip 500 sterilized and forms an extraction pore (fine pore 601) for extracting the tissue fluid on the subject's skin 600 by contacting a fine needle 501 of the fine needle chip 500 with a skin surface of the biological body (subject's skin 600). In a case where the fine pore 601 is formed by the puncture device 400, the fine needle 501 of the fine needle chip 500 has such a size that the fine pore 601 does not reach dermis but stays at epidermis of the skin 600. As shown in FIG. 7, the puncture device 400 comprises a housing 401, a release button 402 provided on a surface of the housing 401, and an array chuck 403 and a spring member 404 which are provided inside the housing 401. An opening (not shown) is formed in a bottom portion 401a of the housing 401. The spring member 404 has a function of biasing the array chuck 403 in a puncturing direction. The array chuck 403 is enabled to be mounted with the fine needle chip 500 at a lower end thereof. Plural fine needles 501 are formed on a lower surface of the fine needle chip 500. Further, the puncture device 400 has a fixing mechanism (not shown) for fixing the array chuck 403 in a state that the array chuck 403 is pushed upward (against a puncturing direction) against a bias of the spring member 404. The user (subject) presses down the release button 402 to release the fixation of the array chuck 403 by the fixing mechanism so that the array chuck 403 moves in the puncturing direction due to the bias of the spring member 404.

[Blood glucose AUC measurement method]

Next, a blood glucose AUC measurement method using the above-described measurement apparatus, sensor chip, and the collection member is explained.

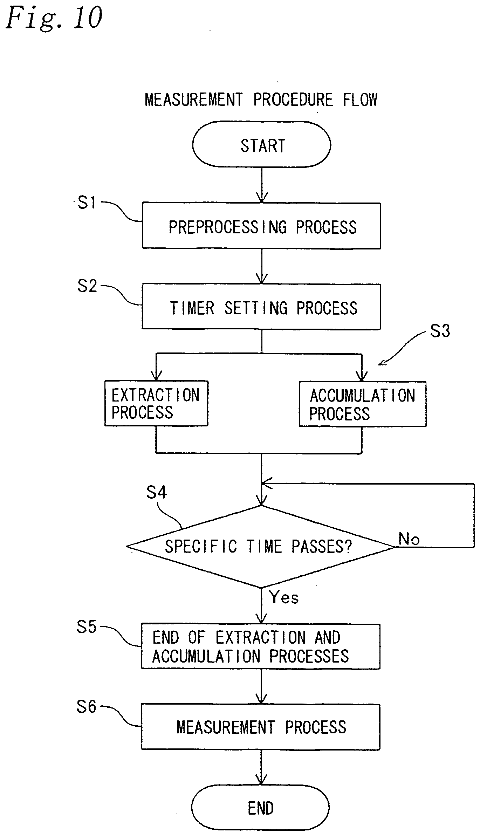

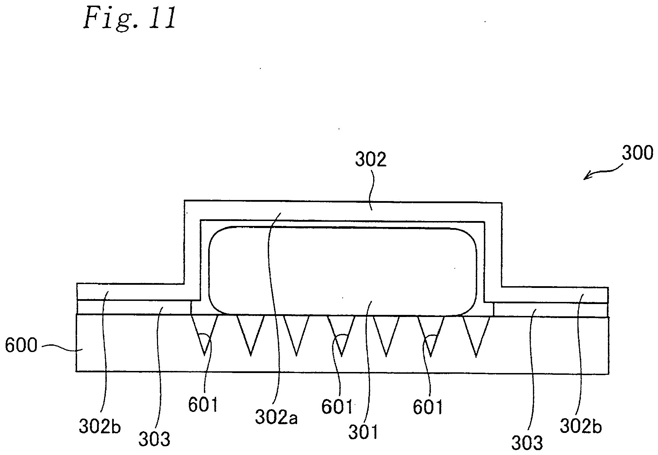

FIG. 10 is a flowchart showing a measurement procedure of the blood glucose AUC measurement method according to one of embodiments of the present invention. FIGs. 11 to 13 are explanatory views of the measurement procedure of this measurement method.

First, with reference to FIG. 10, outline of the measurement procedure of the blood glucose AUC measurement method according to one embodiment of the present invention is explained. Among steps shown in FIG. 10, Steps S1 to S5 are carried out by those practicing the measurement, and Step S6 is carried out by the measurement apparatus 100 of the present embodiment.

First, a site to be measured of the subject is cleaned and fine pores are formed in the site to be measured using a puncture device 400 (Step S1). Next, tissue-fluid extraction time is set up using the timer unit 8 provided in the measurement apparatus 100 (Step S2) . Next, the collection member 300 is fit to the site to be measured, the tissue fluid extraction starts and accumulation of glucose, inorganic ion and others in the tissue fluid starts (Step S3). Next, it is judged whether or not end of the extraction time set up in Step S2 is informed by the alarm device of the timer unit 8 (Step S4) . In a case where it is informed, the collection member 300 is removed and the tissue fluid extraction is finished (Step S5). Next, the collection member 300 finishing extraction is installed in the installation unit 5 of the measurement apparatus 100, the tissue fluid measurement and the blood glucose AUC analysis are carried out (Step S6), and measurement ends.

Hereinafter, respective processes are explained in detail.

(Step S1: Preprocessing Process)

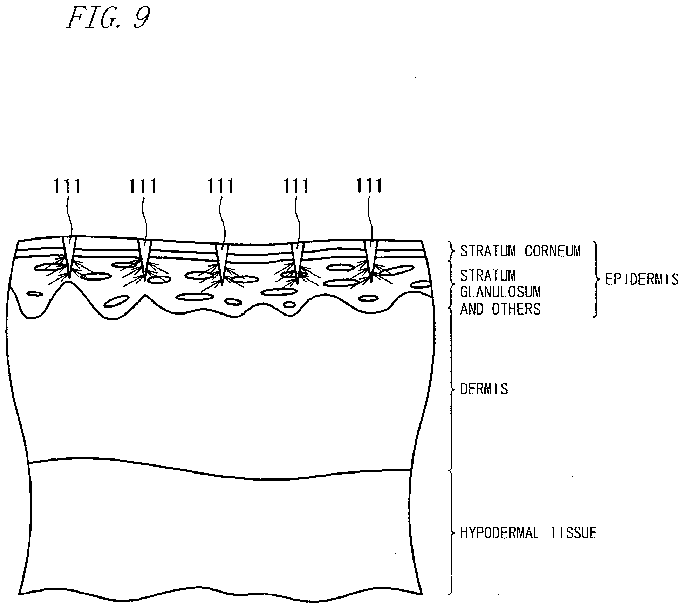

First, the subject cleans a skin 600 with alcohol and others for removing objects (sweat, dust, etc.) to be a disturbing factor for measurement results. After the cleaning, fine pores 601 are formed on the skin 600 with a puncture device 400 (Refer to FIG. 7) mounted with a fine needle chip 500. Specifically, a release button 402 is pressed down in a state that an opening (not shown) of a lower part 401a of the puncture device 400 is disposed on a site where the fine pores 601 of the skin 600 are formed. Thereby engagement of an array chuck 403 with fixing mechanism (not shown) is released, and the array chuck 403 moves to a side of the skin 600 due to bias of a spring member 404. Subsequently, the fine needles 501 of the fine needle chip 500 (Refer to FIG. 8) mounted at a lower end of the array chuck 403 come into contact with the skin 600 of the subject at specific speed. Thus, the fine pores 601 are formed on epidermis portion of the skin 600 of the subject as shown in FIG. 9.

(Step S2: Timer setting process)

Next, the subject sets up time of a timer unit 8 of the measurement apparatus 100 by operating an operation button 7. The setup time is set at, for example, 180 minutes.

(Steps S3 to S5: Extraction-accumulation processes)

Next, as shown in FIG. 11, the subject removes a peel-off paper 304 of the collection member 300 (Refer to FIG. 6) and applies the collection member 300 to the site where the fine pores 601 are formed (Step S3). Thus, a gel 301 contacts with the site where fine pores 601 are formed, and the tissue fluid containing glucose and electrolyte (NaCl) begins to move to the gel 301 through the fine pores 601, and begins the extraction. At the same time with the extraction start, the subject turns on the timer unit 8 of the measurement apparatus 100. Subsequently, the state that the collection member 300 is applied to the skin 600 is kept until the specific time (setup time of the alarm) passes (Step S4). Then, the subject removes the collection member 300 from the skin 600 at time when the alarm sounds after the specific time passes (Step S5). Here since the alarm of the timer unit 8 is set at 180 minutes, the tissue fluid is continuously extracted from the skin for 180 minutes. Thereby the extraction-accumulation process ends.

(Step S6: Measurement process)

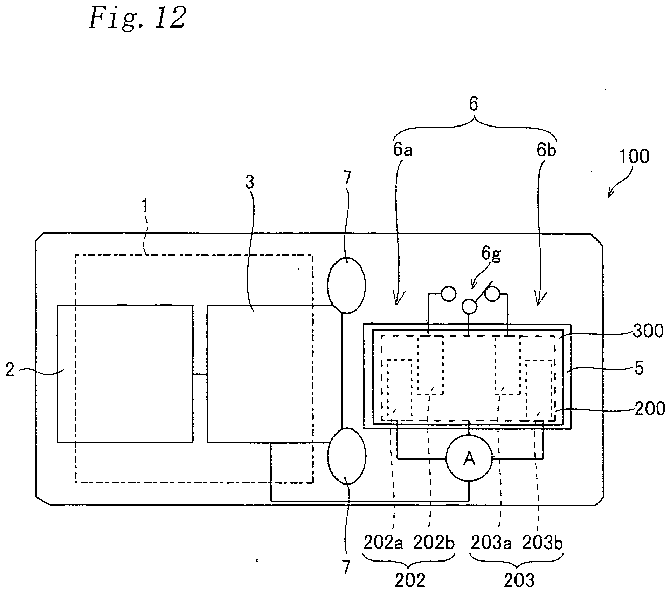

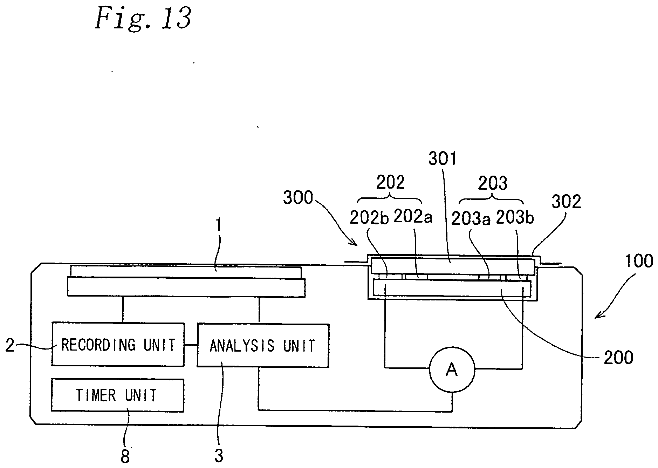

Next, as shown in FIGs. 12 and 13, the subject installs a sensor chip 200 in the installation unit 5 of the measurement apparatus 100 and installs the collection member 300 having finished the extraction on the sensor chip 200. Thus, a first circuit is configured by a glucose concentration measurement circuit 6a of the measurement apparatus 100, a glucose concentration measurement electrode 202 of the sensor chip 200, and a gel 301 of the collection member 300. A second circuit is configured by an ion concentration measurement circuit 6b of the measurement apparatus 100, an ion concentration measurement electrode 203 of the sensor chip 200, and the gel 301 of the collection member 300.

In a case where concentration of the extracted glucose is measured, the subject switches a switch 6g to the glucose concentration measurement circuit 6a by an operation button 7 and instructs start of measurement. Thus, a constant voltage of specific value is applied to the first circuit through a constant voltage control circuit, and a current value of $I_{Glc}$ detected by an ammeter is inputted in an analysis unit 3. Here, the following formula (1) is established between the current value ($I_{Glc}$) and the glucose concentration ($C_{Glc}$) of the gel 301.

$$C_{Glc} = A \times I_{Glc} + B \text{ (A and B are constant numbers) ... (1)}$$

The analysis unit 3 calculates the glucose concentration $C_{Glc}$ from the current value $I_{Glc}$ based on the formula (1).

Further, the analysis unit 3 calculates an extraction glucose quantity ($M_{Glc}$) using thus obtained glucose concentration $G_{Glc}$, extraction solvent quantity, that is, a gel volume V based on the following formula (2).

$$M_{Glc} = C_{Glc} \times V \ ... \ (2)$$

Further, in a case where an extracted inorganic ion concentration is measured, the subject switches a switch 6g to the ion concentration measurement circuit 6b by an operation button 7 and instructs start of measurement. Thus, the constant voltage of specific value is applied to the second circuit through the constant voltage control circuit, and the current value of $I_i$ detected by the ammeter is inputted in the analysis unit 3. Here, the following formula (3) is established between the current value $I_i$ and an ion concentration $C_i$ indicative of the inorganic ion concentration of the gel 301.

$$C_i = C \times I_i + D \ (C \ and \ D \ are \ constant \ numbers) \ ... \ (3)$$

The analysis unit 3 calculates the ion concentration $C_i$ from the current value $I_i$ based on the formula (3).

Further, the analysis unit 3 calculates an extraction rate $J_i$ of inorganic ion at the extraction site from the inorganic ion concentration $C_i$, a volume V of the gel 301, and extraction time t based on the following formula (4).

$$J_i = C_i \times V \times 1/t \ ... \ (4)$$

Then, the analysis unit 3 calculates the predicted glucose permeability ($P_{Glc}$(calc)) indicative of glucose easiness to be extracted from thus calculated ion extraction rate $J_i$ based on the following formula (5).

$$P_{Glc}(calc) = E \times J_i + F \ (E \ and \ F \ are \ constant \ numbers) \ ... \ (5)$$

The formula (5) is obtained as follows.

The glucose permeability indicative of the glucose easiness to be extracted is given by a ratio (this ratio is tentatively referred to as true glucose permeability $P'_{Glc}$) of the blood glucose AUC obtained by blood drawing to an extracted glucose quantity. As described later, because the true glucose permeability $P'_{Glc}$ indicates constant correlation with the ion extraction rate $J_i$, the formula (5) can be obtained by obtaining an approximation formula based on the ion extraction rate $J_i$ and the true glucose permeability $P'_{Glc}$.

According to the formula (5), it is possible to obtain the predicted glucose permeability $P_{Glc}$(calc) indicative of the glucose easiness to be extracted based on ion extraction rate $J_i$ obtainable without conducting the blood drawing.

The analysis unit 3 calculates the predicted blood glucose AUC (predicted $AUC_{BG}$) from the extraction glucose quantity $M_{Glc}$ obtained by the formula (2) and the predicted glucose permeability $P_{Glc}$(calc) obtained by the formula (5), based on the following formula (6).

$$predicted \ AUC_{BG} = M_{Glc} \ / \ P_{Glc}(calc) \ ... \ (6)$$

This predicted blood glucose AUC (predicated $AUC_{BG}$) is a value having high correlation with the blood drawing blood glucose AUC which is calculated by plural times of blood drawing. Here correlativity between the predicated blood glucose AUC and the blood drawing blood glucose AUC is explained later in detail. This predicated blood glucose AUC value is displayed in a display unit 1 and recorded in a recording unit 2. Thus, the measurement process ends.

Further, in the first embodiment, there is exemplified the configuration that glucose concentration $C_{Glc}$, the extraction glucose quantity $M_{Glc}$, the ion concentration $C_i$, the ion extraction rate $J_i$, and the predicated glucose permeability $P_{Glc}$(calc) are calculated to measure the predicted $AUC_{BG}$ in the analysis unit 3. However, other configurations may be employable. For example, the formula (6) for calculating the predicted $AUC_{BG}$ can be replaced with the following formula (6)' by the formulas (1) to (5).

$$\text{Predicted AUC}_{BG} = \{(A \times I_{Glc} + B) \times t\} / [E \times (C \times I_i + D) \times F]$$

$$\dots \ (6)'$$

(A to F are constant numbers)

Therefore, if the formula (6)' is used, it is possible that the analysis unit 3 directly calculates the predicted $AUC_{BG}$ based on the current value $I_{Glc}$ and current value $I_i$.

According to the first embodiment, as described above, since the tissue fluid containing glucose is extracted from the subject's skin for as long as 180 minutes, the tissue fluid containing sufficient quantity of glucose for reflecting a total variable quantity of the glucose in the biological body within the specific period of 180 minutes from the tissue fluid extraction. Therefore, it is possible to measure a value reflecting a total variation quantity of the glucose in the biological body within the specific period, which can not be obtained by the conventional measurement method, by acquiring the predicted blood glucose AUC from the cumulative glucose quantity in the extracted tissue fluid. Further, according to the measurement method of the first embodiment in which no blood drawing is carried out, it is possible to decrease invasiveness degree. Therefore, it is possible to measure the value reflecting a total quantity of glucose circulating in the biological body within the above-described period while decreasing the subject's burden. Further, since the tissue fluid containing glucose is extracted for over 60 minutes and therefore glucose is collected by taking long time, it is possible to extract sufficient quantity of glucose for the measurement without applying a force (e.g. electricity) for collecting glucose from the biological body. Therefore, it is possible to easily perform measurement since a device for applying a force (e.g. electricity) to enhance the collection of glucose is not necessary to be mounted on the subject.

Further, according to the first embodiment, the tissue fluid containing glucose is extracted through the skin 600 with fine pores 601 formed thereof. Therefore, since it becomes easy to extract the tissue fluid through the site where the fine pores 601 are formed in the skin 600, it is easy to collect sufficient quantity of glucose for the measurement without applying a force (e.g. electricity) for collecting the glucose from the biological body.

Here, in the first embodiment, although the time for extracting the tissue fluid is set at 180 minutes, it may not be limited. The time for extracting the tissue fluid may be arbitrarily set at a range of 60 minutes or more. It is useful for grasping clinical conditions to measure an area under the blood glucose curve for 60 minutes after a sugar tolerance and grasp a high blood glucose state, because it is possible to know insulin secretion response rate to the sugar tolerance of the subject. Further, by setting the extraction time at 120 minutes or more, it is possible to grasp the blood glucose variable conditions in longer term than the extraction time of not less than 60 minutes to less than 120 minutes. By setting the extraction time at 180 minutes or more, it is possible to grasp the blood glucose variable conditions in further longer term than that of not less than 60 minutes to less than 180 minutes.

Further, according to the first embodiment, by obtaining the predicted blood glucose AUC corresponding to the blood sampling blood glucose AUC, it is possible to obtain a value corresponding to the blood drawing blood glucose AUC without conducting the blood drawing. Therefore, it is possible to grasp clinical conditions of the diabetes patient while decreasing the subject's burden.

Further, according to the first embodiment, by obtaining the predicted blood glucose AUC based on the quantity of glucose in the extracted tissue fluid and the quantity of inorganic ion in the extracted tissue fluid, it is possible, as described later, to obtain the predicted blood glucose AUC of higher correlativity with the blood sampling blood glucose AUC than electric conductivity based on various types of ion in the tissue fluid is employed. In other words, it is possible to increase accuracy of blood glucose AUC measurement.

Further, according to the first embodiment, by informing the end of extraction by the timer unit 8, it is possible for the subject to know the end of extraction by information of the timer unit 8. Therefore, it is possible to control a difference between the extraction time and the scheduled time.

[Second embodiment]

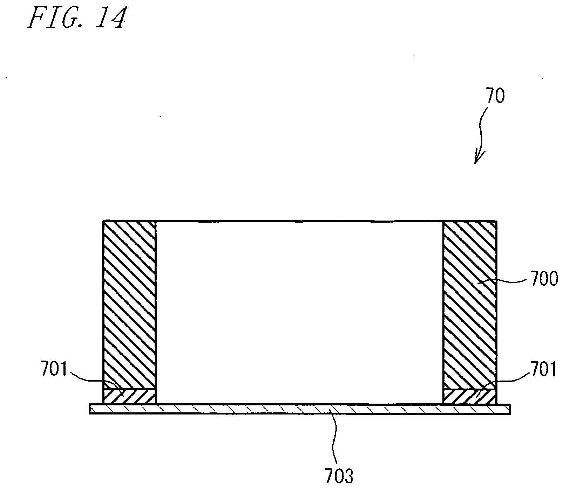

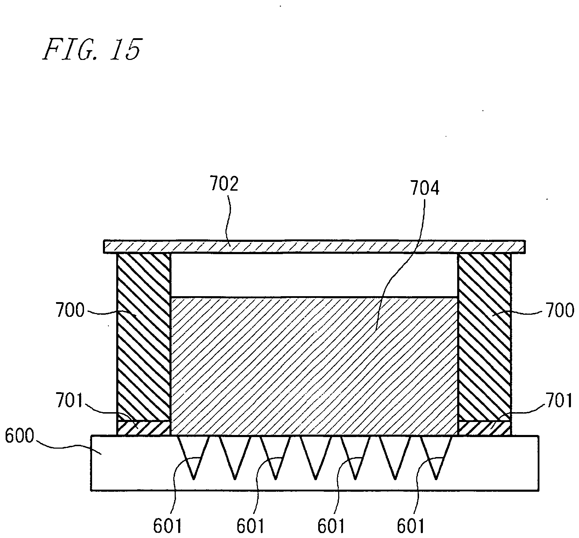

FIGs. 14 and 15 are views for explaining a blood glucose AUC measurement method according to second embodiment of the present invention. This second embodiment is different from the first embodiment in which gel containing pure water as an extraction medium is used. In the second embodiment, an example in which tissue fluid containing glucose and inorganic ion is extracted by using pure water itself is explained. Because a measurement procedure of the second embodiment is substantially same with that of the first embodiment, the second embodiment is explained according to the measurement flow shown in the first embodiment.

As shown in FIG. 14, a reservoir member 70 capable of retaining tissue fluid which is used in the blood glucose AUC measurement method according to the second embodiment comprises a support member 700 which has a short-cylinder shape and has upper and lower openings. An adhesive layer 701 is formed on one end face of the support member 700. Before use, the adhesive layer 701 is applied with a peel-off paper 703.

(Step S1: Preprocessing process)

In the second embodiment, similarly to the first embodiment, first, the subject cleans a skin 600 with alcohol and others for removing objects (sweat, dust, etc.) to be a disturbing factor for measurement results. After the cleaning, fine pores 601 are formed on the skin 600 with a puncture device 400 mounted with a fine needle chip 500.

(Step S2: Timer setting process)

Next, the subject sets up extraction time by a timer unit 8.

(Steps S3 to S5: Extraction-accumulation processes)

Next, as shown in FIG. 15, the subject removes the peel-off paper 703 and applies the support member 700 to a site where the fine pores 601 are formed with the adhesive layer 701. Then a specific quantity of pure water 704 is injected with a pipette (not shown) into the support member 700 through the upper opening. Subsequently the upper opening of the support member 700 is sealed by a seal member 702 for preventing evaporation of the pure water 704. Thus, the pure water 704 contacts with the site where the fine pores 601 are formed, the tissue fluid containing glucose and inorganic ion start moving into the pure water 704 through the fine pores 601, and the extraction starts (Step S3). Further, the subject turns on an alarm device of the timer unit 8 at the same time of extraction start. Subsequently, the state that the support member 700 is applied to the skin 600 is kept until the specific time (setup time of the alarm) passes (Step S4). Then, the subject removes the seal member 702 at the time when the alarm sounds after the specific time passes, and collects the fluid (the pure water 704 extracted of the tissue fluid) in the support member 700 with pipette (Step S5) . Thus, the extraction-accumulation process ends.

(Step S6: Measurement process)

Next, concentration measurement is carried out in the order of inorganic ion concentration and glucose concentration with respect to the collected extraction medium. The inorganic ion concentration is measured using, for example, the ion chromatograph manufactured by Dionex Corporation. An extraction rate $J_i$ of inorganic ion in the extraction site is calculated based on obtained ion concentration $C_i$, a volume V of the extraction medium collected with the pipette, and extraction time t, with the following formula (7).

$$J_i = C_i \times V \times 1/t \ldots (7)$$

Predicted glucose permeability ($P_{Glc}$(calc)) can be obtained from this ion extraction rate $J_i$ with the above-described formula (5) .

Next, glucose concentration $C_{Glc}$ is measured by putting the collected extraction medium in a high-performance liquid chromatography. The extraction glucose quantity $M_{Glc}$ is calculated from the glucose concentration $C_{Glc}$, and Volume V of the used pure water based on the above-described formula (2). Then, the predicted blood glucose AUC (predicted $AUC_{BG}$) is calculated from the extraction glucose quantity $M_{Glc}$ thus obtained and the predicted glucose permeability $P_{Glc}$(calc) based on the above-described formula (6). Thus, the measurement process ends.

<Calculation example>

An example of blood glucose calculation by the measurement method according of the second embodiment is explained. The extraction time is set at 3 hours, and a timer with an alarm function is used as a time information means. Actual measurement values of a subject A used for an experiment are as follows.

Actual measurement value of subject A

Extraction glucose concentration: 3820 ng/ml
Extraction medium (pure water) quantity: 100 $\mu$l
Extraction sodium ion concentration: 2.43 mM

Area under curve (blood drawing measurement method): 281 mg-h/dl

From the formula (2), the extraction glucose concentration $M_{Glc}$ is:

$$M_{Glc} = (\text{Extraction glucose concentration}) \times (\text{Extraction medium quantity})$$

$$= 3820 \times 100/1000$$

$$= 382 \text{ ng}$$

Or from the formula (4), the ion extraction rate $J_i$ is:

$$J_i = (\text{Extraction sodium ion concentration}) \times (\text{Extraction medium quantity}) / (\text{Extraction time})$$

$$= 2.43 \times 10^3 \times 100 \times 10^{-6} / 3$$

$$= 8.1 \times 10^{-2} \ (\mu mol/h)$$

Subsequently, from the formula (5), the predicted glucose permeability $P_{Glc}(calc)$ is:

$$P_{Glc}(calc) = \alpha \times (\text{Ion extraction rate}) + \beta$$

$$= 21.467 \times 8.1 \times 10^{-2} - 0.4198$$

$$= 1.32 \ (10^{-6} \cdot dl/h)$$

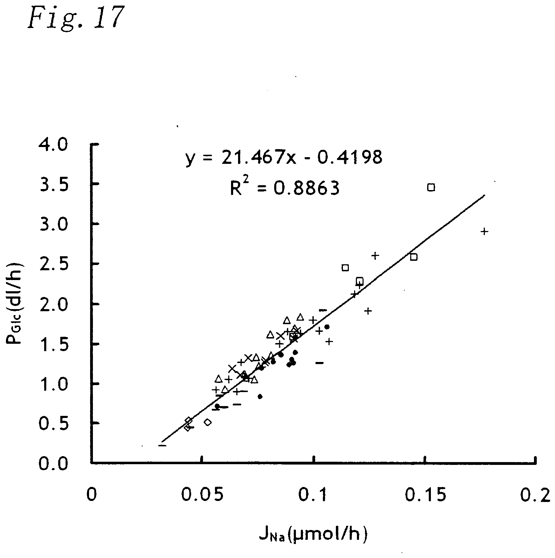

Here, values of $\alpha = 21.467$ and $\beta = -0.4198$ are obtained by an experiment described later with reference to FIG. 17.
Next, the predicted blood glucose AUC (predicted $AUC_{BG}$) is calculated with the above-described formula (6).

$$\text{Predicted } AUC_{BG} = M_{Glc} / P_{Glc}(calc)$$

$$= 382/1.32$$

$$= 289.4 \ (mg \cdot h/dl)$$

The predicted blood glucose AUC (predicted $AUC_{BG}$) thus calculated above matches with a laboratory value of 281 mg·h/dl obtained from the area under curve by blood drawing separately (blood drawing measurement method). This 289.4 (mg-h/dl) is outputted as blood glucose AUC of the subject A. This value is displayed on the display unit 1 of the measurement apparatus 100.
Next, a correlation between the predicted blood glucose AUC (predicted $AUC_{BG}$) actually measured by the measurement method according to the second embodiment and the blood drawing blood glucose AUC ($AUC_{BG}$) is explained with reference to an experiment example. FIGs . 16 to 20 are views explaining the correlation between the predicted blood glucose AUC (predicted $AUC_{BG}$) according to the second embodiment of the present invention and the blood drawing blood glucose AUC ($AUC_{BG}$) . Here, a correlation coefficient $R^2$ is values from -1 to 1 for expressing correlative strength between a vertical axis parameter and a horizontal axis parameter. The value closer to 1 expresses the higher correlation. In a case where respective plots all exist on the same straight line inclining positively, the correlation coefficient is 1.

<Experiment example 1 and comparative experiment example 1>

Prediction accuracy of the blood glucose AUC is verified using pure water as an extraction medium. A sodium ion concentration as a parameter for predicting the glucose permeability is measured by an ion chromatograph. A case where an ion extraction rate obtained based on a value thereof (experiment example 1) is compared with a case where a solvent conductivity is used as a parameter as well (comparative experiment example 1). Experiment conditions are as follows.

[Experiment condition]

Extraction solvent: Pure water 90 $\mu$L
Extraction form: Liquid chamber (Collection member)
Extraction area: 5 mm $\times$ 10 mm
Extraction time: 3 hours
Number of samples (subjects): 7
Number of sites: 66
Glucose concentration measurement method: GOD fluorescence absorbance method
Parameter measurement method: Ion chromatograph (Experiment example 1)
Conductivity meter (Comparative experiment example 1) Fine needle array shape: Length of fine needle = 300 $\mu$m, Number of fine needle = 305 pieces
Puncturing rate: 6 m/s
Blood glucose measurement method: Measurement of forearm SMBG value at 15-minute intervals
Blood glucose AUC measurement method: Calculation based on forearm SMBG value by trapezoidal approximation method

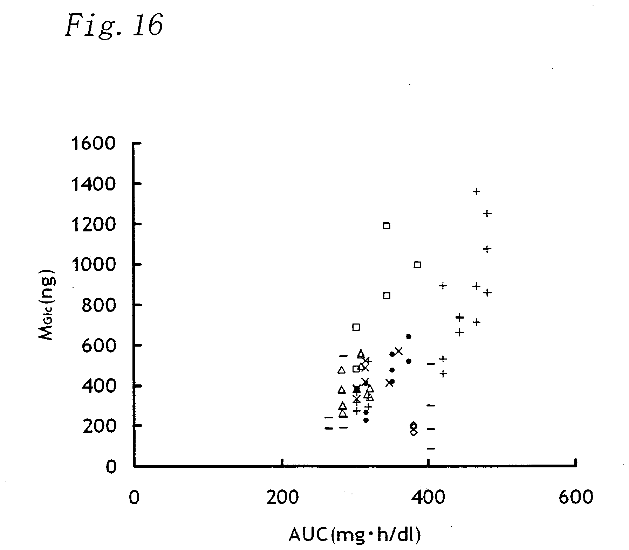

Correlation between a blood glucose AUC ($AUC_{BG}$) obtained by the above-described conditions and an extracted glucose quantity ($M_{Glc}$) is shown in FIG. 16. Here, difference in plot symbols shows difference in subjects in FIG. 16.

As known by FIG. 16, correlativity between the blood glucose AUC ($AUC_{BG}$) and the extracted glucose quantity ($M_{Glc}$) is low. The reason of such low correlativity between both is that glucose permeability (value of division of the extraction glucose quantity by the blood glucose AUC, $M_{Glc}/AUC_{BG}$) is different depending on the subjects and measurement sites.

Next, correlativity between the glucose permeability ($P_{Glc}$) and the ion extraction rate ($J_i$) is studied to predict the glucose permeability ($P_{Glc}$) required for measuring the blood glucose AUC ($AUC_{BG}$).

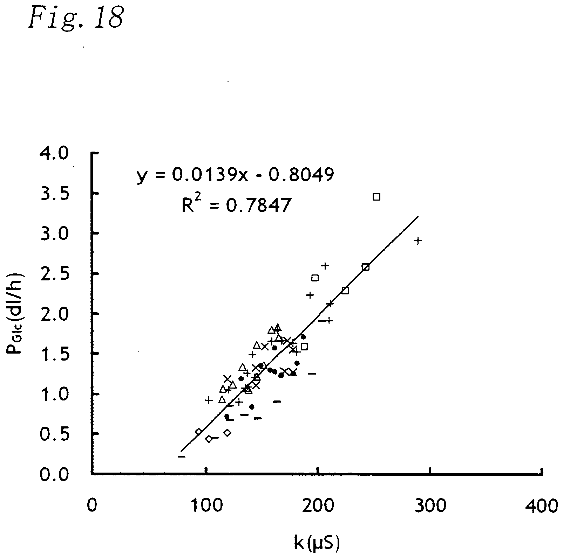

FIG. 17 is a view showing correlativity between the glucose permeability ($P_{Glc}$) and the ion extraction rate ($J_i$) according to Experiment example 1. FIG. 18 is a view showing correlativity between the glucose permeability ($P_{Glc}$) and the solvent conductivity (k) according to Comparative experiment example 1. From FIGs. 17 and 18, it is found that correlation coefficient $R^2$ between the glucose permeability ($P_{Glc}$) and the ion extraction rate ($J_i$) is 0.8863, and it is higher than that between the glucose permeability ($P_{Glc}$) and the solvent conductivity (k) (correlation coefficient $R^2$ = 0.7847).

Then, a predicted glucose permeability ($P_{Glc}$(calc)) can be obtained using this correlativity with the following formula (8) or the formula (9).

$$\text{Experiment example 1: } P_{Glc}(\text{calc}) = \alpha \times J_i + \beta \ (\alpha = 21.467, \ \beta = -0.4198) \ \dots \ (8)$$

$$\text{Comparative experiment example 1: } \alpha \times k + \beta \ (\alpha = 0.0139, \ \beta = -0.8049) \ \dots \ (9)$$

Here, $\alpha$ and $\beta$ are calculation values calculated from the experiment result described above.

The blood glucose AUC ($AUC_{BG}$), the glucose permeability ($P_{Glc}$), and the extraction glucose quantity ($M_{Glc}$) are expressed as the following formula (10).

$$M_{Glc} = P_{Glc} \times AUC_{BG} \dots (10)$$

Therefore, the predicted blood glucose AUC (predicted $AUC_{BG}$) of the respective subjects is calculated using the following formula (11) .

$$\text{predicted } AUC_{BG} = M_{Glc} / P_{Glc}(\text{calc}) \dots (11)$$

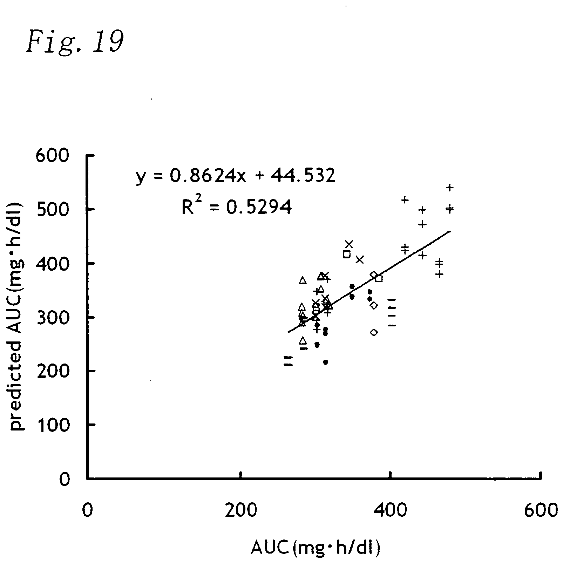

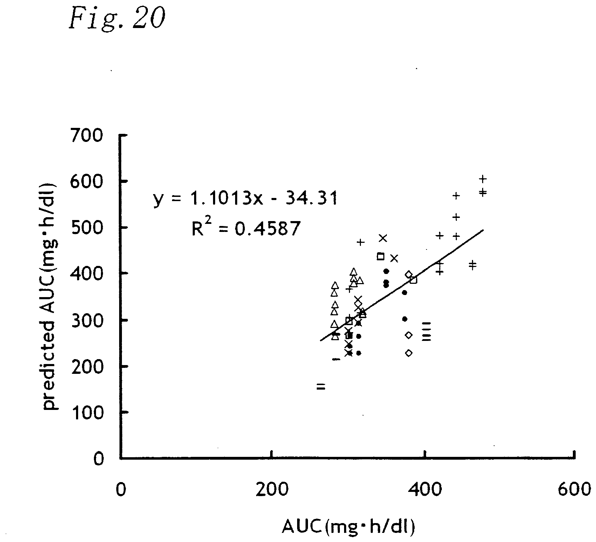

Correlativity between the predicted blood glucose AUC (predicted $AUC_{BG}$) calculated by the formula (11) and the blood drawing blood glucose AUC ($AUC_{BG}$) is shown in FIG. 19 (Experiment example 1) and FIG. 20 (Comparative experiment example 1). As shown in FIGs. 19 and 20, in Comparative experiment example 1 where conductivity is used as a parameter, the blood glucose AUC ($AUC_{BG}$) and the predicted blood glucose AUC (predicted $AUC_{BG}$) have correlativity of about correlation coefficient $R^2 = 0.4587$. Meanwhile, in the Experiment example 1 where the ion extraction rate is used as a parameter, they have higher correlativity of about 0.5294.

Here, in order to evaluate accuracy of the predicted blood glucose AUC (predicted $AUC_{BG}$), a ratio r of the measurement value to the true value is obtained as follows.

$$r = \text{predicted } AUC_{BG} / AUC_{BG}$$

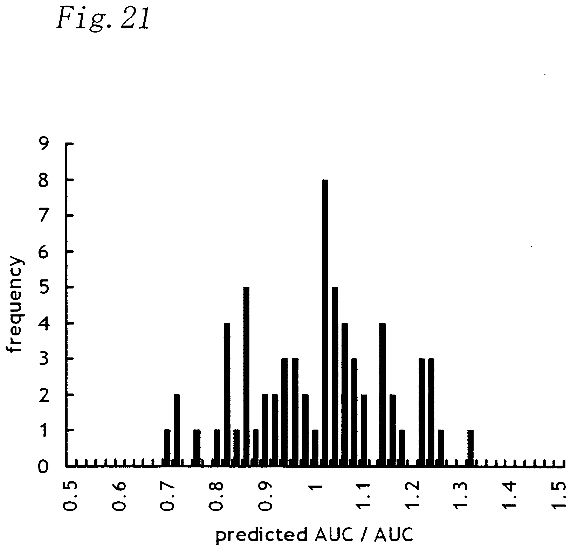

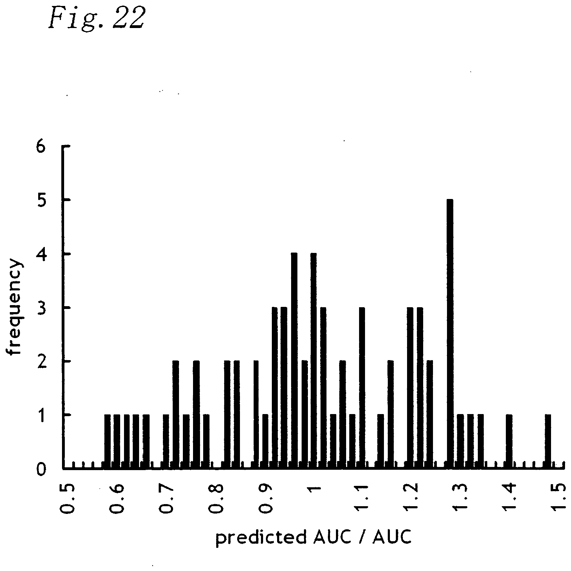

Accuracy of the above-described measurement system is evaluated by evaluating what degree of dispersion this r has around 1 as a center. The dispersion of r in FIG. 19 (Experiment example 1) and FIG. 20 (Comparison experiment example 1) is shown in FIG. 21 and FIG. 22 respectively.

Here, when difference in dispersion of measurement difference in FIGs. 21 and 22 is evaluated by F test, a significant difference of $P < 0.005$ is recognized. In other words, it is found that the ion extraction rate obtained by directly measuring the sodium ion concentration can predict more accurately than the ion extraction rate predicted from the solvent conductivity as a prediction parameter of the glucose permeability predicts and therefore it is useful.

It is imagined that measurement of concentration of single sodium ion which is inorganic ion has higher accuracy of the blood glucose AUC measurement than measurement using all electrolytes including ions other than inorganic ion.

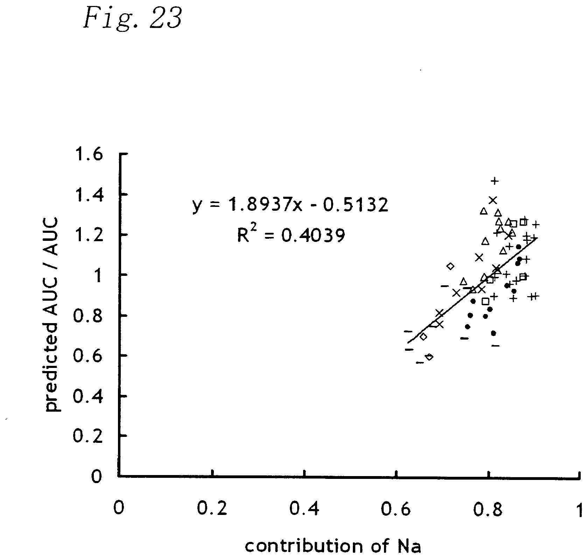

Further, FIG. 23 shows relation between r (rate between measurement value and true value) shown in FIG. 22 and the sodium ion contribution rate in the solvent conductivity at respective measurement points. From FIG. 23, it is found that the subject having high contribution rate of the sodium ion to the conductivity distributes around r = 1 and has higher measurement accuracy of blood glucose AUC compared with the subjects having low contribution rate. Based on this as well, it is imagined that sodium ion which is inorganic ion has good correlation with glucose permeability.

<Experiment example 2>

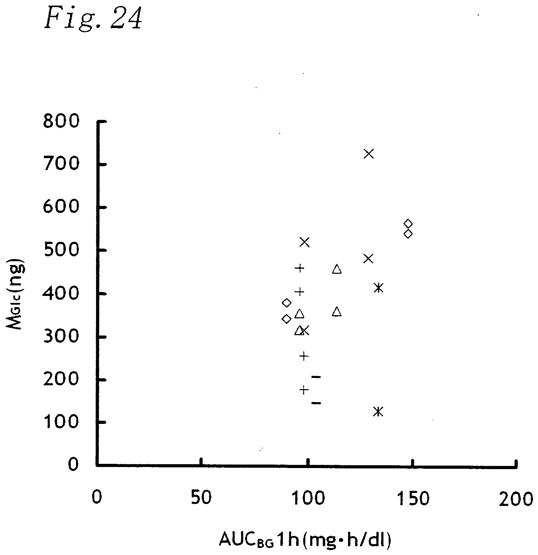

In Experiment example 2, in a case where extraction time is 60 minutes or 120 minutes, it is explained by the following experiment that an area under blood glucose time curve after sugar tolerance is predictable. Here, in FIGs. 24 to 30, difference of plot symbols shows a difference among subjects.

Experiment method is as follows.

[Experiment condition]

Extraction solvent: Pure water 90 μL
Extraction form: Liquid chamber (Collection member)
Extraction area: 5 mm × 10 mm
Extraction time: 60 minutes and 120 minutes
Number of subjects: 6
Number of sites: 22
Glucose measurement method: GOD fluorescence absorbance method
Sodium ion measurement method: Ion chromatograph
Fine needle array shape: Length of fine needle = 300 μm, Number of fine needles = 305 pieces
Puncturing rate: 6 m/s
Blood glucose measurement method: Measurement of forearm SMBG value at 15-minute intervals

Blood glucose AUC measurement method: Calculation based on forearm SMBG value by trapezoidal approximation method

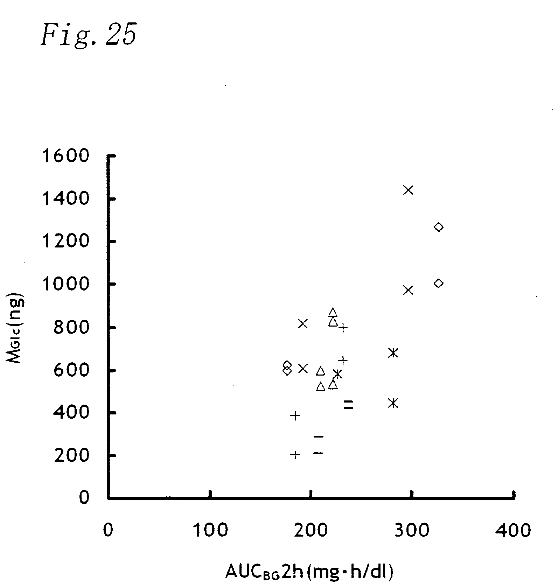

First, prediction value calculation method of $AUC_{BG}1h$ (area under blood glucose time curve 1 hour after the sugar tolerance) and $AUC_{BG}2h$ is shown. A relation between $AUC_{BG}1h$, $AUC_{BG}2h$, and extraction glucose quantity ($M_{Glc}$) is shown in FIGs. 24 and 25.

The following relational formula is established between the extraction glucose ($M_{Glc}$) and $AUC_{BG}xh$ (area under blood glucose curve x hour after sugar tolerance)

$$M_{Glc} = P_{Glc} \times AUC_{BG}xh \ ... \ (12)$$

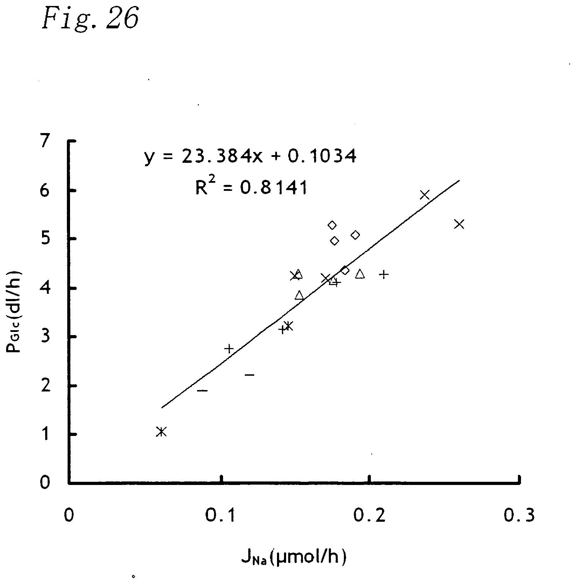

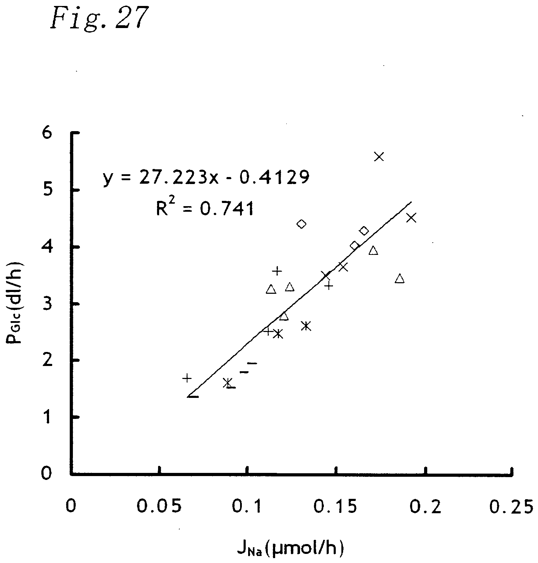

$P_{Glc}$ is a glucose permeability. It is shown that this glucose permeability and an ion extraction rate $J_i$ obtained from a sodium ion concentration of the extraction solvent have correlativity as shown in FIGs. 26 and 27.

A predicted glucose permeability $P_{Glc}$ (calc) of extraction for 1 hour and extraction for 2 hours is obtained from the following formulas (13) and (14).

$$1\text{-hour extraction: } P_{Glc}(calc) = \alpha \times J_i + \beta \ (\alpha = 23.384, \ \beta = 0.1034) \ ... \ (13)$$

$$2\text{-hour extraction: } P_{Glc}(calc) = \alpha \times J_i + \beta \ (\alpha = 27.223, \ \beta = -0.4129) ... \ (14)$$

$AUC_{BG}1h$ and $AUC_{BG}2h$ can be predicted by the following formula (15) being a variation of the formula (12) using $P_{Glc}$(calc) obtained from the formulas (13) and (14).

$$\text{predicted } AUC_{BG} = M_{Glc} \ / \ P_{Glc}(calc) \ ... \ (15)$$

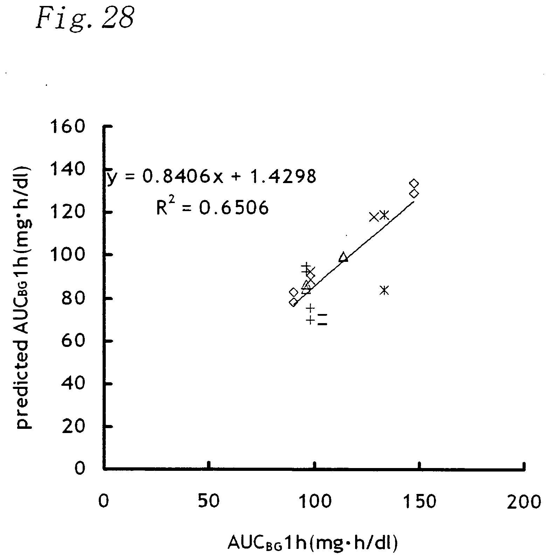

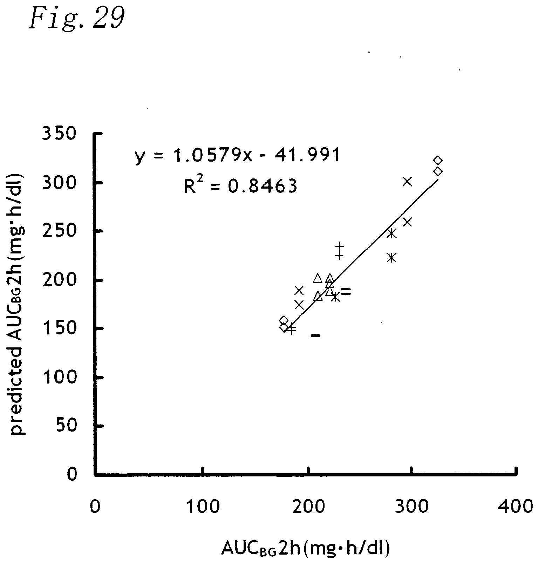

Correlativity between predicted $AUC_{BG}1h$ and predicted $AUC_{BG}2h$ which are obtained from the formula (15) and $AUC_{BG}1h$ and $AUC_{BG}2h$ which are obtained from the blood glucose level is shown in FIGs. 28 and 29.

This result shows that $AUC_{BG}1h$ and $AUC_{BG}2h$ can be measured using the present method because high values of correlation coefficienct $R^2 = 0.6508$ and 0.8463 are obtained.

<Experiment example 3>

Correlativity between glucose permeability ($P_{Glc}$) and an ion extraction rate ($J_i$) at the extraction site is studied using chloride ion concentration as a parameter for predicting the glucose permeability by a method similar to Experiment example 1. Here, measurement of the chloride ion concentration is performed using HPLC. The experiment conditions are as follows.

[Experiment condition]

Extraction solvent: Pure water 90 $\mu$L
Extraction form: Liquid chamber (Collection member)
Extraction area: 5 mm $\times$ 10 mm
Extraction time: 2 hours in which sampling is conducted every ten minutes
Number of subjects: 1
Number of sites: 3
Glucose concentration measurement method: GOD fluorescence absorbance method
Parameter measurement method: Ion chromatograph
Fine needle array shape: Length of fine needle = 300 $\mu$m, Number of fine needles = 305 pieces

Puncturing rate: 6m/s

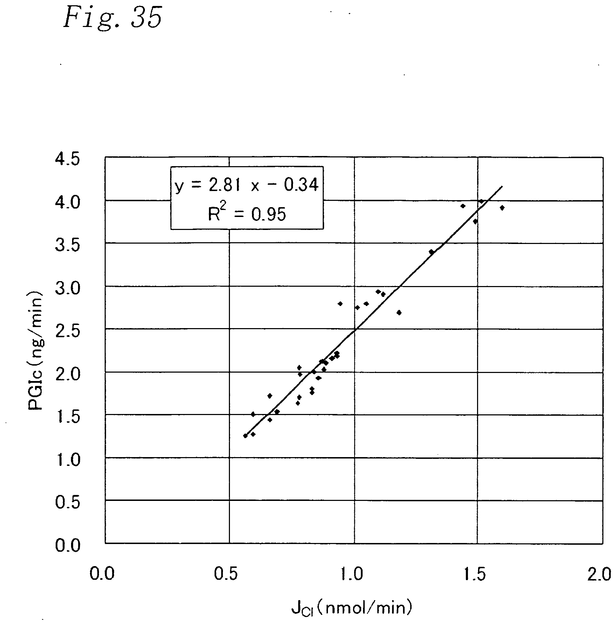

FIG. 35 is a view showing correlativity between the glucose permeability ($P_{Glc}$) and the ion extraction rate ($J_i$) according to Experiment example 3. A correlation coefficient $R^2$ between the glucose permeability ($P_{Glc}$) and the ion extraction rate ($J_i$) is 0.95 and it is found that they have high correlativity. This shows that chloride ion which is inorganic ion can be used as a parameter similarly to the sodium ion.

<Experiment example 4>

Correlativity between glucose permeability ($P_{Glc}$) and an ion extraction rate ($J_i$) at the extraction site is studied using potassium ion concentration as a parameter for predicting the glucose permeability by a method similar to Experiment example 1. Here, measurement of the chloride ion concentration is performed using HPLC. The experiment conditions are as follows.

[Experiment condition]

Extraction solvent: Urea aqueous solution of 4 mol/l (100µL)
Extraction form: Liquid chamber (Collection member)
Extraction area: 5 mm × 10 mm
Extraction time: 2 hours in which sampling is conducted every ten minutes
Number of subjects: 1
Number of sites: 3
Glucose concentration measurement method: GOD fluorescence absorbance method
Parameter measurement method: Ion chromatograph
Fine needle array shape: Length of fine needle = 300 µm, Number of fine needles = 305 pieces
Puncturing rate: 6m/s

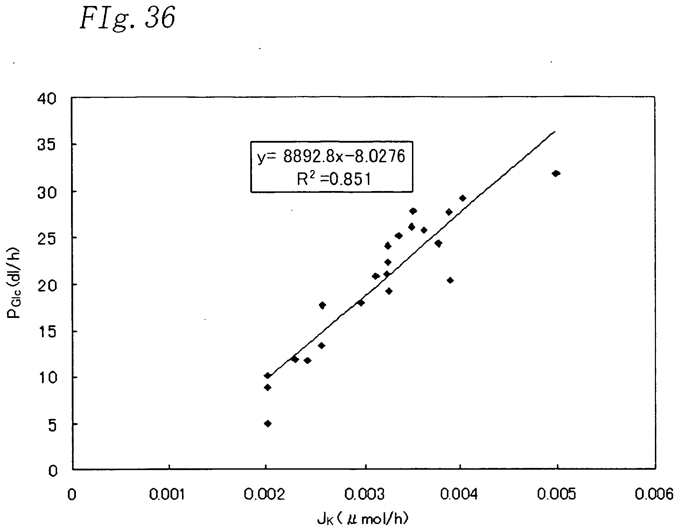

FIG. 36 is a view showing correlativity between the glucose permeability ($P_{Glc}$) and the ion extraction rate ($J_i$) according to Experiment example 4. A correlation coefficient $R^2$ between the glucose permeability ($P_{Glc}$) and the ion extraction rate ($J_i$) is 0.85 and it is found that they have high correlativity. This shows that pottaisum ion which is inorganic ion can be used as a parameter similarly to the sodium ion.

[Other embodiment]

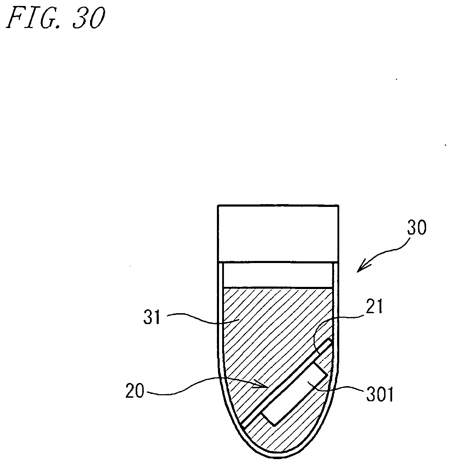

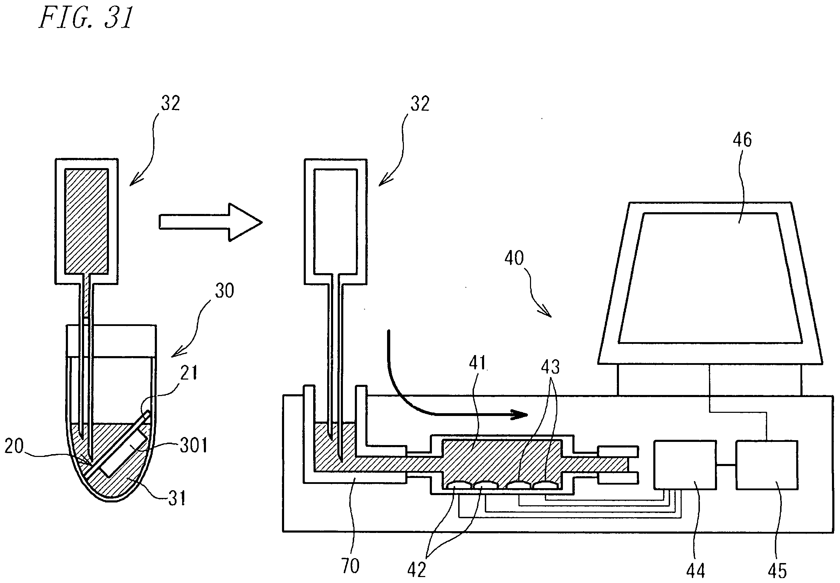

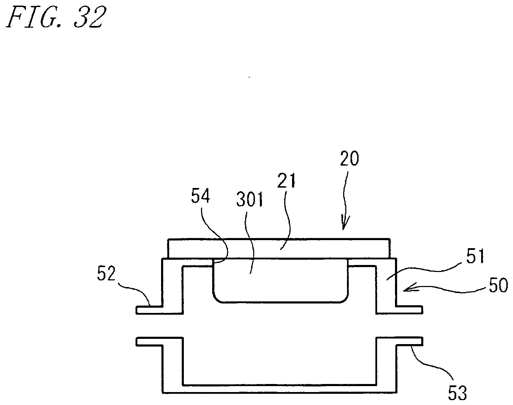

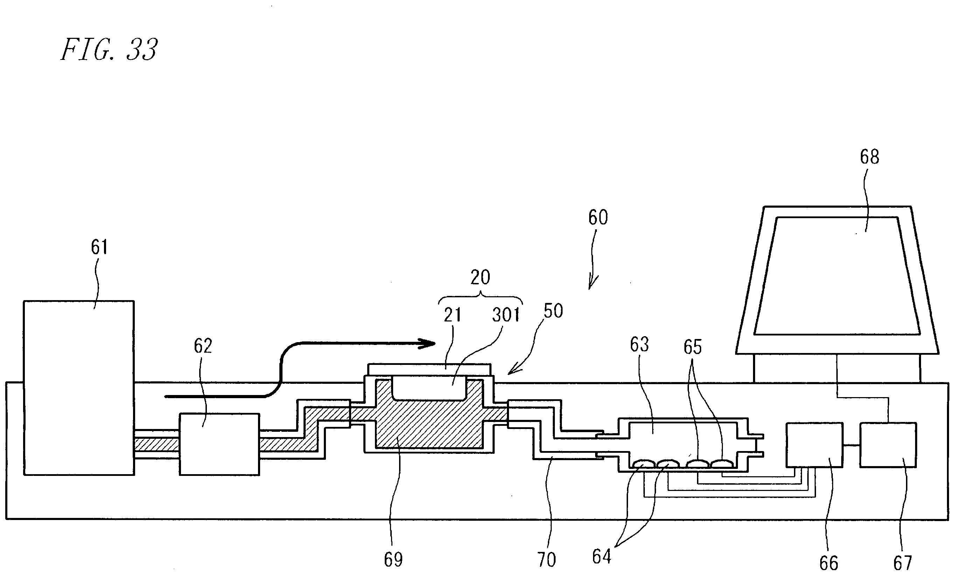

In the measurement method according to the first embodiment, the gel 301 in which the tissue fluid extracted from the body is accumulated is installed in the installation unit 5 of the measurement apparatus 100, and the glucose concentration and the inorganic ion concentration in the gel 301 are measured. However, the analyte in the gel 301 is collected in the pure water in a special container, and analyte concentration of this collection solution may be measured.
For example, as shown in FIG. 30, a gel reservoir 20 (the gel 301 is disposed on one surface of the substrate 21) having the gel 301 which has finished extraction of analyte from the skin is immersed in a collection fluid 31 formed of pure water in a collection tube 30, and the analyte accumulated in the gel 301 is collected. After collection of the analyte ends, the collection fluid 31 in the collection tube 30 is moved to a measurement unit 41 in a measurement system 40 via an introduction portion 70 through a syringe 32, as shown in FIG. 31. In the measurement unit 41, glucose concentration measurement electrodes 42 and ion concentration measurement electrodes 43 which are similar to the above-described measurement apparatus 100 are arranged, a glucose concentration and an inorganic ion concentration are measured by an electric control unit 44 and an analysis unit 45 by the above-described method using the formulas (1) to (6), and blood glucose AUC is analyzed. The obtained result is outputted in a display unit 46.
Further, the analyte in the gel 301 may be collected by the other method. As shown in FIG. 32, the gel reservoir 20 having the gel 301 which has finished extraction of the analyte from the skin is set in a special collection cartridge 50. This collection cartridge 50 is formed of a cartridge main body 51 in a box shape, and an inlet 52 of the collection fluid is formed on one of wall surfaces of the cartridge main body 51 which oppose to each other, and an outlet 53 of the collection fluid is formed on the other. The gel reservoir 20 is set to the collection cartridge 50 in such a manner that the gel 301 is projected into the cartridge main body 51 from an opening 54 formed on one surface of the cartridge main body 51.
Next, as shown in FIG. 33, the collection cartridge 50 is set in the specific place of the measurement apparatus 60. This measurement apparatus 60 includes a tank unit 61 and a pump unit 62, and a flow passage as an introduction portion 70 for collection fluid is formed to a measurement unit 63 through the tank unit 61, the pump unit 62, and the cartridge main body 51. Further, glucose concentration measurement electrodes 64 and ion concentration measurement electrodes 65 are arranged in the measurement unit 63 similarly to the above-described measurement system 100. After the collection cartridge 50 is set, a collection fluid 69, contained in the tank unit 61, for collecting the analyte in the gel is moved into the cartridge main body 51 by driving the pump unit 62 (Refer to FIG. 33). Further, although illustration is omitted, a valve is arranged on downstream side of the outlet 53 of the cartridge main body 51, and the valve is closed before the collection fluid 69 is transported into the cartridge main body 51.

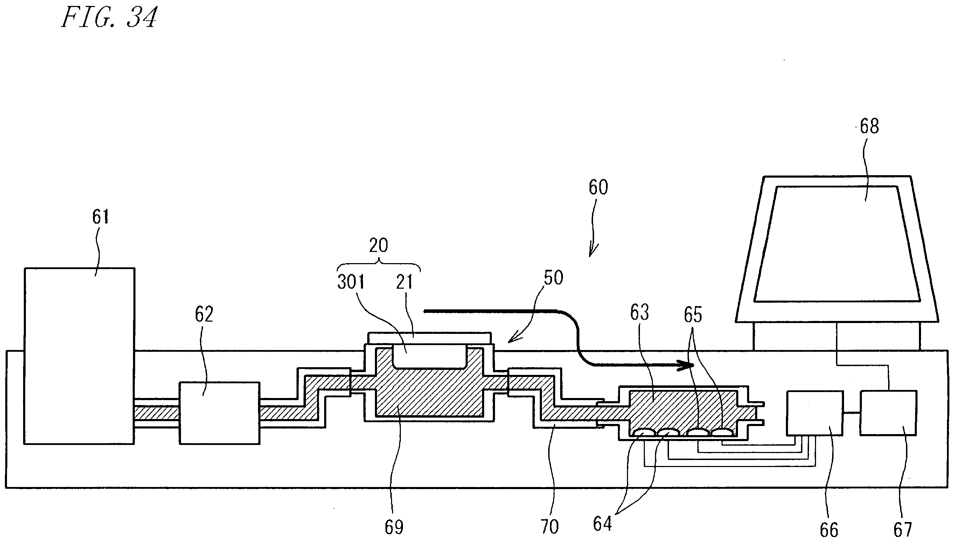

While the state of the cartridge main body 51 filled up with the collection fluid 69 is suspended for a given time, the analyte in the gel 301 is collected in the collection fluid 69. Subsequently, the valve is opened and the collection fluid 69 is transported from the cartridge main body 51 to the measurement unit 63 via the flow passage being an introduction portion 70 by driving the pump unit 62, as shown in FIG. 34. Next, the glucose concentration and the inorganic ion concentration are measured by an electric control unit 66 and an analysis unit 67 by the above-described method using the formulas (1) to (6), and the blood glucose AUC is analyzed. Thus obtained result is outputted on a display unit 68.

Meanwhile, it should be considered that the embodiments and experiment examples disclosed here are not limited but all exemplification. A scope of the present invention is not shown by the above-described embodiments and experiment examples but by a scope of claims. Further it includes means equal to the scope of claims and all modification within the scope.

For example, in the first embodiment and the second embodiment, it is exemplified that the tissue fluid is extracted from the skin by passive diffusion without electric application. However, the present invention is not limited to this but the tissue fluid may be extracted due to electric power by an iontophoresis method. Even in this case, in a case where it takes long time over 60 minutes for extraction, high voltage application for conducting extraction in a short time is not required. Therefore, a device for applying electricity is made small.

Further, in the examples of the first embodiment and the second embodiment, the tissue fluid is extracted after the fine pores 601 are formed by the puncture device 400. However, the present invention is not limited to this, but the tissue fluid may be extracted without forming fine pores. Or the extraction of the tissue fluid may be enhanced by removing the corneum of skin such as pealing, instead of forming fine pores. In a case where the fine pores are not formed, the extraction of the tissue fluid may be enhanced by iontophoresis and others.

Further, in the first embodiment, using the gel made of polyvinyl alcohol is exemplified as the gel 301. However the present invention is not limited to this, but a gel made of cellulose or polyacrylic acid may be used.

Further, in the examples of the first embodiment and the second embodiment, the predicted blood glucose AUC is calculated as a value corresponding to a blood drawing blood glucose AUC being one of indexes used for grasping clinical conditions of the diabetes patients. However the present invention is not limited to this, but a value obtained using the measurement method of the present invention may be used for grasping clinical conditions of other disease.

Further, in the examples of the first embodiment and the second embodiment, glucose quantity in the tissue fluid is measured. However, the present invention is not limited to this but a quantity of objects other than glucose which is included in the tissue fluid may be measured and used as any index. As objects being measured by the present invention, there are, for example, biochemical components and drugs administrated to the subject. Example of the biochemical component are, for example, albumin, globulin and enzyme of protein which is one type of biochemical components. Further, example of biochemical components other than protein are, for example, creatinine, creatine, urinary acid, amino acid, fructose, galactose, pentose, glycogen, lactic acid, pyruvic acid, and ketone body. Further, examples of drug are, for example, digitalis preparation, theophylline, arrhythmic drug, antiepileptic drug, aminoglycoside antibiotic, glycopeptide biotic, antithrombotic drug, and immune suppressant drug.

Further, in the example of the first embodiment, the value of calculated predicted blood glucose AUC is displayed as it is on the display unit 1. However the present invention is not limited to this, but a value of the calculated predicted blood glucose AUC divided by extraction time may be displayed on the display unit 1. Therefore, even in a case of different extraction time, it is possible to easily compare those values because predicted blood glucose AUC per time unit is enabled to obtain.

Further, there are shown Experiment examples 1 to 4 is which sodium ion, potassium ion and chloride ion are used as inorganic ion. However, inorganic ion usable in the present invention is not limited thereto. Here, inorganic ion usable in the present invention is not particularly limited as long as it is contained in the tissue fluid. Example of such inorganic ion are, for example, sodium ion, potassium ion, chloride ion, calcium ion, magnesium ion, ammonium ion, nitrite ion, nitrate ion and phosphate ion. Among these, sodium ion, potassium ion and chloride ion are preferable.

## Claims

1. An in vivo component measurement method comprising steps of:

forming (S1) fine pores on a skin of a biological body so as to enhance the extraction of a tissue fluid from the biological body,

extracting (S3) the tissue fluid from the biological body into an extraction medium and accumulating (S3) an objective component and an inorganic ion in the extracted tissue fluid, wherein the tissue fluid is extracted through the skin where the fine pores are formed;

informing an end of extraction at a predetermined time;

acquiring (S6) a concentration of the accumulated inorganic ion at the predetermined time;

acquiring (S6) a concentration of the accumulated objective component at the predetermined time; predicting (S6) an area under blood concentration time curve of the objective component based on the concentration of the accumulated inorganic ion and the concentration of the accumulated objective component.

2. The in vivo component measurement method according to claim 1,
   wherein the objective component is glucose.

3. The in vivo component measurement method according to claim 1,
   wherein the predetermined time is 60 minutes or more.

4. The in vivo component measurement method according to any one of claims 1 or 3,
   wherein the predetermined time is 180 minutes or more.

5. The in vivo component measurement method according to any one of claims 1 to 4,
   wherein the inorganic ion is sodium ion, potassium ion or chloride ion.

6. An in vivo component measurement apparatus comprising:

   a puncture device (400) configured to form fine extraction pores in a biological body;
   setting unit (5) for setting a collection member arranged to accumulate an objective component and an inorganic ion contained in a tissue fluid extracted from a biological body;
   a detection unit (200, 6a, 6b, 42, 43) for acquiring a concentration of the objective component and a concentration of the inorganic ion in the collection member set in the setting unit (5), wherein the objective component and the inorganic ion are accumulated by the collection member at a predetermined time; and
   an analysis unit (3) for predicting an area under a blood concentration time curve of the objective component based on the concentration of the inorganic ion and the concentration of the objective component in the collection member.

7. The in vivo component measurement apparatus according to claim 6,
   wherein the predetermined time is 60 minutes or more.

8. The in vivo component measurement apparatus according to claim 6 or 7,
   wherein the predetermined time is 180 minutes or more.

9. The in vivo component measurement apparatus according to any one of claims 6 to 8,
   wherein the objective component is glucose.

10. The in vivo component measurement apparatus according to any one of claims 6 to 9, further comprising:
    a time information unit (8) for informing that the predetermined time has passed after start of the extraction of the tissue fluid.

11. The in vivo component measurement apparatus according to any one of claims 6 to 10,
    wherein the detection unit (200, 6a, 6b, 42, 43) includes a first detection unit (6a) for acquiring the concentration of the objective component and a second detection unit (6b) for acquiring the concentration of the inorganic ion.

12. The in vivo component measurement apparatus according to any one of claims 6 to 11,
    wherein the detection unit (200, 6a, 6b, 42, 43) includes a glucose concentration measurement electrode (42) having a work electrode with a glucose oxidase enzyme membrane formed on a platinum electrode and a counter electrode formed of a platinum electrode.

13. The in vivo component measurement apparatus according to any one of claims 6 to 12,

wherein the detection unit (200, 6a, 6b, 42, 43) includes an ion concentration measurement electrode (43) having an ion selective electrode made of silver/silver chloride and has a selection membrane for inorganic ion, and a counter electrode formed of silver/silver chloride.

14. The in vivo component measurement apparatus according to any one of claims 6 to 13,
wherein the inorganic ion is sodium ion, potassium ion or chloride ion.

**Patentansprüche**

1. In-vivo-Komponenten-Messverfahren, umfassend die Schritte:

Bilden (S1) von feinen Poren auf einer Haut von einem biologischen Körper, um die Extraktion von einem Gewebefluid aus dem biologischen Körper zu erhöhen,
Extrahieren (S3) des Gewebefluids aus dem biologischen Körper in ein Extraktionsmedium und Sammeln (S3) einer Zielkomponente und eines anorganischen Ions im extrahierten Gewebefluid, wobei das Gewebefluid durch die Haut extrahiert wird, wo die feinen Poren gebildet sind;
Informieren über ein Ende der Extraktion bei einer vorbestimmten Zeit;
Erfassen (S6) einer Konzentration vom angesammelten anorganischen Ion bei der vorbestimmten Zeit;
Erfassen (S6) einer Konzentration von der angesammelten Zielkomponente bei der vorbestimmten Zeit;
Vorhersagen (S6) einer Fläche unter der Blutkonzentration-Zeitkurve der Zielkomponente, basierend auf der Konzentration vom angesammelten anorganischen Ion und der Konzentration der angesammelten Zielkomponente.

2. In-vivo-Komponenten-Messverfahren nach Anspruch 1,
wobei die Zielkomponente Glucose ist.

3. In-vivo-Komponenten-Messverfahren nach Anspruch 1,
wobei die vorbestimmte Zeit 60 Minuten oder mehr ist.

4. In-vivo-Komponenten-Messverfahren nach einem der Ansprüche 1 oder 3,
wobei die vorbestimmte Zeit 180 Minuten oder mehr ist.

5. In-vivo-Komponenten-Messverfahren nach einem der Ansprüche 1 bis 4,
wobei das anorganische Ion ein Natriumion, Kaliumion oder Chloridion ist.

6. In-vivo-Komponenten-Messeinrichtung, umfassend:

eine Punktionsvorrichtung (400), ausgelegt zum Bilden feiner Extraktionsporen in einem biologischen Körper;
Einstelleinheit (5) zum Einstellen eines Sammelbauteils, angeordnet zum Sammeln einer Zielkomponente und eines anorganischen Ions, enthalten in einem aus einem biologischen Körper extrahierten Gewebefluid;
eine Detektionseinheit (200, 6a, 6b, 42, 43) zum Erfassen einer Konzentration von der Zielkomponente und einer Konzentration vom anorganischen Ion im Sammelbauteil, das in der Einstelleinheit (5) angepasst wird, wobei die Zielkomponente und das anorganische Ion durch das Sammelbauteil bei einer vorbestimmten Zeit gesammelt werden; und
eine Analyseneinheit (3) zum Vorhersagen einer Fläche unter einer Blutkonzentration-Zeitkurve der Zielkomponente, basierend auf der Konzentration vom anorganischen Ion und der Konzentration der Zielkomponente im Sammelbauteil.

7. In-vivo-Komponenten-Messeinrichtung nach Anspruch 6,
wobei die vorbestimmte Zeit 60 Minuten oder mehr ist.

8. In-vivo-Komponenten-Messeinrichtung nach Anspruch 6 oder 7,
wobei die vorbestimmte Zeit 180 Minuten oder mehr ist.

9. In-vivo-Komponenten-Messeinrichtung nach einem der Ansprüche 6 bis 8,
wobei die Zielkomponente Glucose ist.

**10.** In-vivo-Komponenten-Messeinrichtung nach einem der Ansprüche 6 bis 9, weiter umfassend:
eine Zeitinformationseinheit (8) zum Informieren, dass nach dem Beginn der Extraktion vom Gewebefluid die vorherbestimmte Zeit vergangen ist.

**11.** In-vivo-Komponenten-Messeinrichtung nach einem der Ansprüche 6 bis 10,
wobei die Detektionseinheit (200, 6a, 6b, 42, 43) eine erste Detektionseinheit (6a) zum Erfassen der Konzentration von der Zielkomponente und einer zweiten Detektionseinheit (6b) zum Erfassen der Konzentration vom anorganischen Ion aufweist.

**12.** In-vivo-Komponenten-Messeinrichtung nach einem der Ansprüche 6 bis 11,
wobei die Detektionseinheit (200, 6a, 6b, 42, 43) eine Glucosekonzentration-Messelektrode (42) mit einer Arbeitselektrode mit einer Glucoseoxidaseenzymmembran, gebildet auf einer Platinelektrode, und eine Gegenelektrode, gebildet von einer Platinelektrode, aufweist.

**13.** In-vivo-Komponenten-Messeinrichtung nach einem der Ansprüche 6 bis 12,
wobei die Detektionseinheit (200, 6a, 6b, 42, 43) eine Ionenkonzentration-Messelektrode (43) mit einer ionenselektiven Elektrode, hergestellt aus Silber/Silberchlorid, aufweist und eine Selektionsmembran für anorganisches Ion und eine Gegenelektrode, gebildet aus Silber/Silberchlorid, aufweist.

**14.** In vivo-Komponenten-Messeinrichtung nach einem der Ansprüche 6 bis 13,
wobei das anorganische Ion ein Natriumion, Kaliumion oder Chloridion ist.

## Revendications

**1.** Procédé de mesure de composant in vivo comprenant les étapes de :

formation (S1) de pores fins sur une peau d'un corps biologique de manière à améliorer l'extraction d'un fluide tissulaire du corps biologique,
extraction (S3) du fluide tissulaire du corps biologique dans un milieu d'extraction et accumulation (S3) d'un composant objectif et d'un ion inorganique dans le fluide tissulaire extrait, dans lequel le fluide tissulaire est extrait à travers la peau où sont formés les pores fins ;
information d'une fin d'extraction à un temps prédéterminé ;
acquisition (S6) d'une concentration de l'ion inorganique accumulé au temps prédéterminé ;
acquisition (S6) d'une concentration du composant objectif accumulé au temps prédéterminé ;
prédiction (S6) d'une zone sous une courbe de temps de concentration de sang du composant objectif sur la base de la concentration de l'ion inorganique accumulé et de la concentration du composant objectif accumulé.

**2.** Procédé de mesure de composant in vivo selon la revendication 1,
dans lequel le composant objectif est le glucose.

**3.** Procédé de mesure de composant in vivo selon la revendication 1,
dans lequel le temps prédéterminé est 60 minutes ou plus.

**4.** Procédé de mesure de composant in vivo selon l'une quelconque des revendications 1 ou 3,
dans lequel le temps prédéterminé est 180 minutes ou plus.

**5.** Procédé de mesure de composant in vivo selon l'une quelconque des revendications 1 à 4,
dans lequel l'ion inorganique est l'ion sodium, l'ion potassium ou l'ion chlorure.

**6.** Appareil de mesure de composant in vivo comprenant :

un dispositif de perforation (400) configuré pour former des pores d'extraction fins dans un corps biologique ;
une unité de pose (5) pour poser un élément de collecte agencé pour accumuler un composant objectif et un ion inorganique contenus dans un fluide tissulaire extrait du corps biologique ;
une unité de détection (200, 6a, 6b, 42, 43) pour acquérir une concentration du composant objectif et une concentration de l'ion inorganique dans l'élément de collecte posé dans l'unité de pose (5), dans lequel le composant objectif et l'ion inorganique sont accumulés par l'élément de collecte à un temps prédéterminé ; et

une unité d'analyse (3) pour prédire une zone sous une courbe de temps de concentration de sang du composant objectif sur la base de la concentration de l'ion inorganique et de la concentration du composant objectif dans l'élément de collecte.

7. Appareil de mesure de composant in vivo selon la revendication 6,
dans lequel le temps prédéterminé est 60 minutes ou plus.

8. Appareil de mesure de composant in vivo selon la revendication 6 ou 7,
dans lequel le temps prédéterminé est 180 minutes ou plus.

9. Appareil de mesure de composant in vivo selon l'une quelconque des revendications 6 à 8,
dans lequel le composant objectif est le glucose.

10. Appareil de mesure de composant in vivo selon l'une quelconque des revendications 6 à 9, comprenant en outre :
une unité d'informations de temps (8) pour informer que le temps prédéterminé s'est écoulé après le début de l'extraction du fluide tissulaire.

11. Appareil de mesure de composant in vivo selon l'une quelconque des revendications 6 à 10,
dans lequel l'unité de détection (200, 6a, 6b, 42, 43) inclut une première unité de détection (6a) pour acquérir la concentration du composant objectif et une seconde unité de détection (6b) pour acquérir la concentration de l'ion inorganique.

12. Appareil de mesure de composant in vivo selon l'une quelconque des revendications 6 à 11,
dans lequel l'unité de détection (200, 6a, 6b, 42, 43) inclut une électrode de mesure de concentration de glucose (42) ayant une électrode de travail avec une membrane à enzyme de glucose oxydase formée sur une électrode de platine et une contre-électrode formée d'une électrode de platine.

13. Appareil de mesure de composant in vivo selon l'une quelconque des revendications 6 à 12,
dans lequel l'unité de détection (200, 6a, 6b, 42, 43) inclut une électrode de mesure de concentration d'ion (43) ayant une électrode sélective d'ion composée d'argent/chlorure d'argent et a une membrane de sélection pour ion inorganique, et une contre-électrode formée d'argent/chlorure d'argent.

14. Appareil de mesure de composant in vivo selon l'une quelconque des revendications 6 à 13,
dans lequel l'ion inorganique est l'ion sodium, l'ion potassium ou l'ion chlorure.

*Fig. 1*

Fig. 2

*Fig.3*

*Fig. 4*

*Fig. 5*

200

202

202b 202a

203

203a 203b

201

*Fig. 6*

EP 2 198 774 B1

*Fig. 7*

26

Fig. 8

*FIG. 9*

111    111    111    111    111

} STRATUM CORNEUM ]
} STRATUM GLANULOSUM AND OTHERS    } EPIDERMIS

} DERMIS

} HYPODERMAL TISSUE

*Fig. 10*

MEASUREMENT PROCEDURE FLOW

START

S1 — PREPROCESSING PROCESS

S2 — TIMER SETTING PROCESS

S3

EXTRACTION PROCESS

ACCUMULATION PROCESS

S4 — SPECIFIC TIME PASSES? — No

Yes

S5 — END OF EXTRACTION AND ACCUMULATION PROCESSES

S6 — MEASUREMENT PROCESS

END

*Fig. 11*

*Fig. 12*

*Fig. 13*

FIG. 14

FIG. 15

*Fig. 16*

Fig. 17

*Fig. 18*

*Fig. 19*

*Fig. 20*

*Fig. 21*

*Fig. 22*

*Fig. 23*

*Fig. 24*

*Fig. 25*

*Fig. 26*

## Fig. 27

$$y = 27.223x - 0.4129$$
$$R^2 = 0.741$$

Y-axis: $P_{Glc}(dl/h)$

X-axis: $J_{Na}(\mu mol/h)$

Fig. 28

*Fig. 29*

*FIG. 30*

FIG. 31

EP 2 198 774 B1

FIG. 32

*FIG. 33*

*FIG. 34*

*Fig. 35*

*FIg. 36*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9502357 A
- US 20070232875 A
- EP 1964512 A2
- US 2007027383 A1